(19) Europäisches Patentamt
European Patent Office
Office européen des brevets

(11) EP 1 418 176 B1

(12) # EUROPÄISCHE PATENTSCHRIFT

(45) Veröffentlichungstag und Bekanntmachung des Hinweises auf die Patenterteilung:
**28.04.2010 Patentblatt 2010/17**

(51) Int Cl.:
***C07D 405/04*** $^{(2006.01)}$ ***A61K 31/415*** $^{(2006.01)}$

(21) Anmeldenummer: **03028260.2**

(22) Anmeldetag: **30.09.1998**

(54) **Pyrazol-Derivate, ihre Herstellung und ihre Verwendung in Arzneimitteln**

Pyrazole derivates, their preparation and their use in drugs

Dérivés de pyrazole, leur préparation et leur utilisation dans les médicaments

(84) Benannte Vertragsstaaten:
**AT BE CH CY DE DK ES FI FR GB GR IE IT LI LU NL PT SE**

(30) Priorität: **06.10.1997 DE 19744026**

(43) Veröffentlichungstag der Anmeldung:
**12.05.2004 Patentblatt 2004/20**

(62) Dokumentnummer(n) der früheren Anmeldung(en) nach Art. 76 EPÜ:
**98118494.8 / 0 908 456**

(73) Patentinhaber: **Sanofi-Aventis Deutschland GmbH**
**65929 Frankfurt am Main (DE)**

(72) Erfinder:
- **Schindler, Ursula, Dr.**
  **65812 Bad Soden (DE)**
- **Schönafinger, Karl, Dr.**
  **63755 Alzenau (DE)**
- **Strobel, Hartmut, Dr.**
  **65835 Liederbach (DE)**

(56) Entgegenhaltungen:
**EP-A- 0 667 345 WO-A-98/16507**

- **CHEMICAL ABSTRACTS, vol. 125, no. 3, 15. Juli 1996 (1996-07-15) Columbus, Ohio, US; abstract no. 33633, Seite 903; XP002056085 & CN 111 926 A (YONGXIN PHARMCEUTICAL INDUSTRY)**
- **YU S -M ET AL: "INHIBITION OF PLATELET FUNCTION BY A02131-1, A NOVEL INHIBITOR OF CGMP-SPECIFIC PHOSPHODIESTERASE, IN VITRO AND IN VIVO" BLOOD, Bd. 87, Nr. 9, 1. Mai 1996 (1996-05-01), Seiten 3758-3767, XP002056082**
- **WU CH -CH ET AL: "GAMMAC-1 INHIBITED HUMAN PLATELET AGGREGATION THROUGH NO-INDEPENDENT ACTIVATION OF SOLUBLE GUANYLATE CYCLASE" BRITISH JOURNAL OF PHARMACOLOGY, Bd. 116, Nr. 3, 1995, Seiten 1973-1978, XP002056083**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 418 176 B1

Printed by Jouve, 75001 PARIS (FR)

**Beschreibung**

Die vorliegende Erfindung betrifft Pyrazol-Derivate der Formel I,

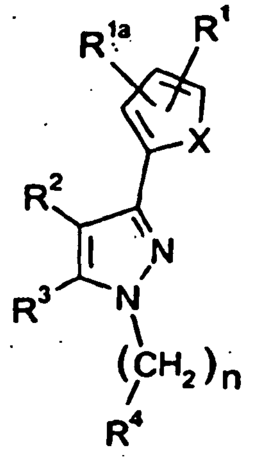

I

in der X, $R^1$, $R^{1a}$, $R^2$, $R^3$, $R^4$ und n die unten angegebenen Bedeutungen haben, die wertvolle Arzneimittelwirkstoffe zur Therapie und Prophylaxe von Krankheiten sind, zum Beispiel von Herz-Kreislauferkrankungen wie Bluthochdruck, Angina pectoris, Herzinsuffizienz, Thrombosen oder Atherosklerose. Die Verbindungen der Formel I haben die Fähigkeit zur Modulation der körpereigenen Produktion von cyclischem Guanosinmonophosphat (cGMP) und eignen sich generell zur Therapie und Prophylaxe von Krankheitszuständen, die mit einem gestörten cGMP-Haushalt verbunden sind. Die Erfindung betrifft weiterhin Verfahren zur Herstellung von Verbindungen der Formel I, ihre Verwendung zur Therapie und Prophylaxe der bezeichneten Krankheitszustände und zur Herstellung von Arzneimitteln dafür, sowie pharmazeutische Präparate, die Verbindungen der Formel I enthalten.

cGMP ist ein wichtiger intrazellulärer Botenstoff, der über die Modulation cGMPabhängiger Proteinkinasen, Phosphodiesterasen und Ionen kanälen eine Vielzahl verschiedener Effekte auslöst. Beispiele sind die Glattmuskelrelaxation, die Inhibition der Thrombozytenaktivierung und die Hemmung der Glattmuskelzellproliferation und der Leukozytenadhäsion. cGMP wird durch partikuläre und lösliche Guanylatcyclasen (GC) als Antwort auf eine Reihe extra- und intrazellulärer Stimuli produziert. Im Falle der partikulären Guanylatcyclasen erfolgt die Stimulation im wesentlichen durch peptidische Signalstoffe, wie dem atrialen natriuretischen Peptid oder dem cerebralen natriuretischen Peptid. Die löslichen Guanylatcyclasen (sGC), bei denen es sich um cytosolische, heterodimere Hämproteine handelt, werden dagegen im wesentlichen durch eine Familie niedermolekularer, enzymatisch gebildeter Faktoren reguliert. Wichtigstes Stimulans ist das Stickstoffmonoxid (NO) oder eine nahe verwandte Spezies. Die Bedeutung anderer Faktoren wie Kohlenmonoxid oder dem Hydroxylradikal ist noch weitgehend ungeklärt. Als Aktivierungsmechanismus der Aktivierung durch NO wird die Anbindung von NO an das Häm unter Ausbildung eines pentakoordinierten Häm-Nitrosyl-Komplexes diskutiert. Die damit verbundene Freisetzung des im Basal-Zustand an das Eisen gebundenen Histidins überführt das Enzym in die aktivierte Konformation.

Aktive lösliche Guanylatcyclasen setzen sich aus je einer $\alpha$- und einer $\beta$-Untereinheit zusammen. Von den Untereinheiten wurden mehrere Subtypen beschrieben, die sich untereinander bezüglich Sequenz, gewebespezifischer Verteilung und Expression in verschiedenen Entwicklungsstadien unterscheiden. Die Subtypen $\alpha$, und $\beta$, werden hauptsächlich in Gehirn und Lunge exprimiert, während $\beta_2$ vor allem in Leber und Niere gefunden wird. In humanem fötalen Gehirn konnte der Subtyp $\alpha_2$ nachgewiesen werden. Die als $\alpha_3$ und $\beta$, bezeichneten Untereinheiten wurden aus menschlichem Gehirn isoliert und sind homolog zu $\alpha_1$ und $\beta_1$ Neuere Arbeiten weisen auf eine $\alpha_{2i}$-Untereinheit hin, die ein Insert in der katalytischen Domäne enthält. Alle Untereinheiten zeigen große Homologien im Bereich der katalytischen Domäne. Die Enzyme enthalten vermutlich ein Häm pro Heterodimer, das über $\beta_1$-Cys-78 und/oder $\beta_1$-His-105 gebunden ist und Teil des regulatorischen Zentrums ist.

Unter pathologischen Bedingungen kann die Bildung Guanylatcyclase-aktivierender Faktoren vermindert sein oder es kann durch das vermehrte Auftreten freier Radikale ein verstärkter Abbau derselben erfolgen. Die daraus resultierende verminderte Aktivierung der sGC führt über die Abschwächung der jeweiligen cGMP-vermittelten Zellantwort beispielsweise zum Anstieg des Blutdrucks, zur Plättchenaktivierung oder zu vermehrter Zellproliferation und Zelladhäsion. Als Folge kommt es zur Ausbildung von endothelialer Dysfunktion, Atherosklerose, Bluthochdruck, stabiler und instabiler Angina pectoris, Thrombosen, Myocardinfarkt, Schlaganfällen oder erektiler Dysfunktion. Die pharmakologische Stimulation der sGC bietet eine Möglichkeit zur Normalisierung der cGMP-Produktion und erlaubt damit die Behandlung bzw. Prävention derartiger Krankheiten.

Zur pharmakologischen Stimulation der sGC wurden bisher fast ausschließlich Verbindungen verwendet, deren Wirkung auf einer intermediären NO-Freisetzung beruht, beispielsweise organische Nitrate. Der Nachteil dieser Behandlungsweise liegt in der Toleranzentwicklung und Wirkungsabschwächung und der deshalb erforderlich werdenden höheren Dosierung.

Verschiedene nicht über eine NO-Freisetzung wirkende sGC-Stimulatoren wurden von Veseley in einer größeren Zahl von Arbeiten beschrieben. Die Verbindungen, bei denen es sich zumeist um Hormone, Pflanzenhormone, Vitamine oder zum Beispiel Naturstoffe wie Echsengifte handelt, zeigen jedoch durchweg nur schwache Effekte auf die cGMP-Bildung in Zellysaten (D. L. Veseley, Eur. J. Clin. Invest. 15 (1985) 258; D. L. Veseley, Biochem. Biophys. Res. Comm. 88 (1979) 1244). Eine Stimulation Häm-freier Guanylatcyclase durch Protoporphyrin IX wurde durch Ignarro et al. (Adv. Pharmacol. 26 (1994) 35) nachgewiesen. Pettibone et al. (Eur. J. Pharmacol. 116 (1985) 307) beschrieben für Diphenyliodoniumhexafluorophosphat eine blutdrucksenkende Wirkung und führten diese auf eine Stimulation der sGC zurück. Isoliquiritiginin, das an isolierten Rattenaorten eine relaxierende Wirkung zeigt, aktiviert laut Yu et al. (Brit. J. Pharmacol. 114 (1995) 1587) ebenfalls die sGC. Ko et al. (Blood 84 (1994) 4226), Yu et al. (Biochem. J. 306 (1995) 787) und Teng et al. (Brit. J. Pharmacol. 116 (1995) 1973) wiesen eine sGC-stimulierende Aktivität von 1-Benzyl-3-(5-hydroxymethyl-2-furyl)-indazol nach und demonstrierten eine antiproliferative und thrombozytenhemmende Wirkung. Verschiedene Indazole werden in EP-A-667 345 als Inhibitoren der Thrombozytenaggregation beschrieben.

Überraschend wurde nun gefunden, daß die Pyrazol-Derivate der Formel I eine Guanylatcyclase-Aktivierung bewirken und damit für die Therapie und Prophylaxe von Krankheiten geeignet sind, die mit einem niedrigen cGMP-Spiegel verbunden sind.

Die vorliegende Erfindung betrifft somit Verbindungen der Formel I,

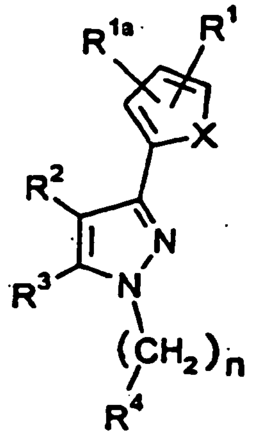

I

in der

X für O steht;

$R^1$ für einen ungesättigten Alkylrest mit bis zu 10 Kohlenstoffatomen steht, der eine oder zwei Doppelbindungen oder eine oder zwei Dreifachbindungen oder eine Doppelbindung und eine Dreifachbindung enthält und der durch Hydroxy substituiert ist;

$R^{1a}$ für Wasserstoff steht; .

$R^2$ und $R^3$ zusammen mit den sie tragenden Kohlenstoffatomen einen Benzolring bilden, der unsubstituiert oder durch einen oder mehrere gleiche oder verschiedene Substituenten $R^5$ substituiert sein kann, wobei $R^5$ für Halogen, $(C_1-C_3)$-Alkyl, $CF_3$ oder $(C_1-C_3)$-Alkyl-O- steht;

$R^4$ für Phenyl oder 5- oder 6-gliedriges Heteroaryl steht, die jeweils unsubstituiert oder durch einen oder mehrere Substituenten aus der Reihe Halogen, $(C_1-C_3)$-Alkyl und $CF_3$ substituiert sein können:

n für 0, 1 oder 2 steht;

in allen ihren stereoisomeren Formen und Mischungen davon in allen Verhältnissen, und ihre physiologisch verträglichen Salze.

Alkylreste können geradkettig oder verzweigt sein. Dies gilt auch, wenn sie substituiert sind oder wenn sie in anderen Gruppen enthalten sind, zum Beispiel in Alkoxygruppen. Beispiele für Alkylgruppen sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, n-Heptyl, n-Octyl, n-Nonyl und n-Decyl. Im Fall der Alkylgruppe, die für $R^1$ steht oder In $R^1$ enthalten ist, sind unter dem Begriff Alkyl ungesättigte Alkylreste zu verstehen, die eine oder zwei Doppelbindungen oder eine oder zwei Dreifachbindungen oder eine Doppelbindung und eine Dreifachbindung enthalten. Beispiele für solche Reste sind der Vinylrest, der 2-Propenylrest (Allylrest), der 2-Butenylrest, der 3-Methyl-2-butenylrest, der Ethinylrest, der 2-Propinylrest (Propargylrest) oder der 3-Butinylrest.

Phenylreste können unsubstituiert oder einfach oder mehrfach, zum Beispiel zweifach oder dreifach, substituiert sein, wobei sich die Substituenten in beliebigen Positionen befinden können. Monosubstituierte Phenylreste können in der 2-Position, der 3-Position oder der 4-Position substituiert sein, disubstituierte Phenylreste in der 2,3-Position, der 2,4-Position, der 2,5-Position, der 2,6-Position, der 3,4-Position oder der 3,5-Position. In trisubstituierten Phenylresten können sich die Substituenten beispielsweise in 2,3,4-Position, 2,3,5-Position, 2,3,6-Position oder 3,4,5-Position befinden.

Beispiele für heterocyclische 5-Ring- und 6-Ring-Systeme, von denen sich die aromatischen, 5- oder 6-gliedrige Gruppe Heteroaryl, die unsubstituiert oder substituiert sein kann, ableiten kann, sind Pyrrol, Furan, Thiophen, Imidazol, Pyrazol, 1,2,3-Triazol, 1,2,4-Triazol, 1,2-Oxazol, 1,3-Thiazol, 1,2-Thiazol, Tetrazol, Pyridin, Pyridazin, Pyrimidin, Pyrazin, 1,2,3-Triazin, 1,2,4-Triazin, 1,3,5- Triazin, 1,2,4,5-Tetrazin, alle jeweils in maximal ungesättigter Form (aromatischer Form). Der heterocyclische Rest kann über ein beliebiges Kohlenstoffatom gebunden sein, er kann also zum Beispiel im Fall von Resten, die sich vom Furansystem, dem Thiophensystem oder dem Pyrrolsystem ableiten, in der 2-Position oder der 3-Position gebunden sein, im Fall von Resten, die sich vom Imidazolsystem ableiten oder vom 1,3-Thiazolsystem ableiten, in der 2- Position, der 4-Position oder der 5-Position, oder im Fall von Resten, die sich vom Pyridinsystem ableiten, in der 2-Position, der 3-Position oder der 4-Position. Stickstoffheterocyclen, die an einem Ring-Stickstoffatom einen Substituenten tragen können, können auch über ein Ring-Stickstoffatom gebunden sein, wenn der betreffende heterocyclische Rest an ein Kohlenstoffatom gebunden ist. Die Heterocyclen können einfach oder mehrfach, zum Beispiel zweifach, dreifach oder vierfach, und in beliebigen Positionen substituiert sein. Substituenten an einem Heterocyclus können auch einen Ring bilden, es können also kondensierte Heterocyclen vorliegen, zum Beispiel cyclopenta-kondensierte, cyclohexa-kondensierte oder benzo-kondensierte Heterocyclen. Als Substituenten an einem Stickstoffatom eines Heterocyclus kommen insbesondere zum Beispiel ($C_1$-$C_3$)-Alkylreste in Betracht. Stickstoffheterocyclen können auch als N-Oxide vorliegen.

Halogen bedeutet, sofern nicht anders angegeben, Fluor, Chlor, Brom oder Iod, vorzugsweise Fluor oder Chlor.

Wenn der von $R^2$ und $R^3$ zusammen mit den sie tragenden Kohlenstoffatomen gebildete Benzolring durch einen oder mehrere Reste $R^5$ substituiert ist, so ist er bevorzugt durch einen, zwei, drei oder vier gleiche oder verschiedene Reste $R^5$ substituiert, besonders bevorzugt durch einen oder zwei Reste. Die Reste $R^5$ können sich in beliebigen Positionen befinden.

Der Substituent $R^1$ kann sich in beliebigen Positionen des Heterocyclus befinden, also in den Positionen 3, 4 und 5 des Furanrings, In den Verbindungen der Formel I bildern $R^2$ und $R^3$ zusammen mit den sie tragenden Kohlenstoffatomen einen Benzolring, so daß kondensierte Pyrazole vorliegen, und zwar Benzo-Pyrazole (Formel Ia), die auch als Indazole bezeichnet werden (die erfindungsgemäßen Verbindungen sind 1H-Indazole). In den Verbindungen der Formel Ia haben X, $R^1$, $R^{1a}$, $R^4$, $R^5$ und n die oben angegebenen Bedeutungen und y steht entsprechend den obigen Angaben für 0 oder eine ganze Zahl, bevorzugt für 0, 1, 2, 3 oder 4, besonders bevorzugt für 0, t oder 2. Die Reste $R^5$, die gleich oder verschieden sein können, können sich in beliebigen Positionen im Carbocyclus befinden.

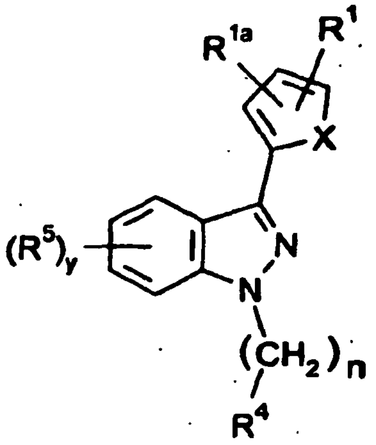

Ia

Steht n in der Formel I für die Zahl 0, so ist der Rest $R^4$ direkt an den das Pyrazol-Stickstoffatom gebunden.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als links- als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Die Trennung eines Stereoisomerengemisches kann auf der Stufe der Verbindungen der Formel I erfolgen oder im Verlaufe der Synthese.

Bei Vorliegen von beweglichen Wasserstoffatomen umfaßt die vorliegende Erfindung auch alle tautomeren Formen der Verbindungen der Formel I, zum Beispiel Lactam/Lactim-Tautomere.

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die eine oder mehrere saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren. Verbindungen der Formel I, die eine oder mehrere basische, das heißt protonierbare, Gruppen enthalten, können in Form ihrer Säureadditionssalze mit anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäuere, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel I, zum Beispiel Hydrate oder Addukte mit Alkoholen, sowie Derivate der Verbindungen der Formel I, zum Beispiel Ester, Pro-Drugs und Metabolite, die wie die Verbindungen der Formel I wirken.

$R^1$ steht bevorzugt für CH(OH)-Alk, worin Alk für einen ungesättigten Alkylrest steht, der bis zu 5 Kohlenstoffatome

enthält und die Merkmale aufweist, die oben für einen für $R^1$ stehenden oder in $R^1$ enthaltenen Alkylrest angegeben sind. Besonders bevorzugt befindet sich der Rest $R^1$ in der 5-Position der Heterocyclus.

n steht bevorzugt für 0 oder 1.

In den Resten $R^2$ und $R^3$ vorkommende Reste $R^5$ stehen bevorzugt für $CF_3$.

Ein 5- oder 6-gliedriger Heteroarylrest bedeutet bevorzugt den Rest eines aromatischen Heterocyclus mit einem, zwei, drei oder vier Heteroatomen aus der Reihe Stickstoff, Sauerstoff und Schwefel, besonders bevorzugt mit einem oder zwei Heteroatomen oder den Tetrazolylrest. Ganz besonders bevorzugt bedeutet ein 5-oder 6-gliedriger Heteroarylrest den Rest-eines der aromatischen Heterocyclen Furan, Thiophen, 1,3-Thiazol, 1,3-Oxazol, 1,2-Oxazol, Tetrazol, Pyridin und pyrimidin, darüber hinaus bevorzugt des 1,3-Thiazols oder des Tetrazols. Diese Reste sind über ein Kohlenstoffatom gebunden sind und können unsubstituiert oder wie oben angegeben substituiert sein.

Bevorzugte Verbindungen der Formel I sind solche, in denen einer oder mehrere der darin enthaltenen Reste bevorzugte Bedeutungen haben, wobei alte Kombinationen von bevorzugten Substituentendefinitionen umfaßt werden. Auch von allen bevorzugten Verbindungen der Formel I umfaßt die vorliegende Erfindung alle ihre stereoisomeren Formen und Mischungen davon in allen Verhältnissen, und ihre physiologisch verträglichen Salze.

Die Verbindungen der Formel I können nach verschiedenen Verfahren hergestellt werden, die im folgenden beschrieben sind und die ebenfalls Gegenstand der vorliegenden Erfindung sind.

Die erfindungsgemäßen Verbindungen der Formel I, in der die Reste $R^2$ und $R^3$ zusammen mit den sie tragenden Kohlenstoffatomen einen Benzolring bilden, also Verbindungen der Formel Ia, können dadurch dargestellt werden, daß Verbindungen der Formel VII mit Hydrazinen der Formel III oder deren Salzen umgesetzt werden. In den Formeln VII, VIII und Ia' können die Reste X, $R^{1'}$, $R^{1a'}$, $R^{4'}$, $R^{5'}$ und die Zahl n die oben angegebenen Bedeutungen von X, $R^1$, $R^{1a}$, $R^4$, $R^5$ und n haben, in diesen Resten können aber auch funktionelle Gruppen in geschützter Form bzw. in Form von Vorstufen vorliegen. y hat in den Formeln Ia', VII und VIII die oben angegebenen Bedeutungen, y steht kann hier also für 0, 1, 2, 3 oder 4 stehen. $Z^1$ in den Formeln VII und VIII steht für eine Abgangsgruppe, zum Beispiel für Halogen oder für andere geeignete Gruppen wie den Trifluormethansulfonyloxyrest. Bevorzugt steht $Z^1$ für Fluor. Aus den Verbindungen der Formel Ia' können dann gegebenenfalls erfindungsgemäße Verbindungen der Formel Ia erhalten werden, indem man in einem anschließenden Reaktionsschritt die in geschützter Form bzw. in Form von Vorstufen vorliegenden Gruppen in die gewünschten, in den obigen Definitionen von $R^1$, $R^4$ und $R^5$ genannten funktionellen Gruppen überführt.

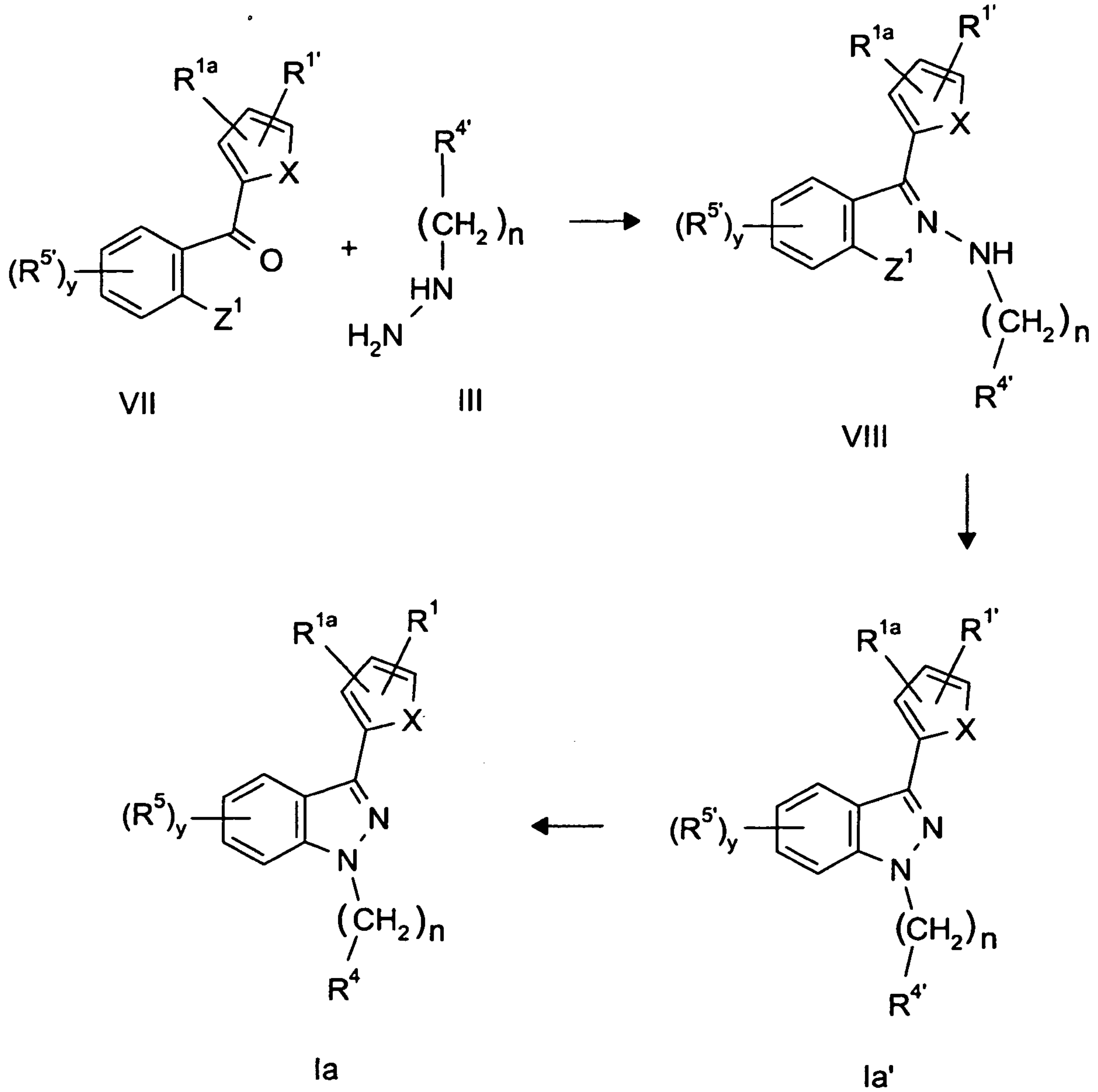

VII III VIII Ia Ia'

Bei der Umsetzung von Verbindungen der Formeln VII und III kann als Zwischenprodukt zunächst ein Hydrazon entstehen, also eine Verbindung der Formel VIII, das je nach der Reaktivität der eingesetzten Ausgangssubstanzen und den Reaktionsbedingungen isolierbar sein kann. Je nach dem Einzelfall kann es günstig sein, die Umsetzung der Verbindungen der Formeln III und VII durch die Wahl der Reaktionsbedingungen wie Lösungsmittel, Temperatur und Katalysator so zu führen, daß sie zunächst nur zum Hydrazon der Formel VIII führt, und dieses dann in einem separaten Schritt zum Indazol der Formel Ia' zu cyclisieren, es kann aber auch günstig sein, die Reaktion so zu führen, daß direkt das Indazol entsteht. In anderen Fällen wiederum kann es aufgrund der Reaktivitäten der Ausgangsverbindungen angebracht sein, die Reaktion in zwei Stufen durchzuführen und in der zweiten Stufe, der Cyclisierung des Hydrazons der Formel VIII zum Indazol der Formel Ia', die Reaktionsbedingungen abzuändern. So ist es zum Beispiel, wenn die Gruppe $Z^1$ in der Verbindung der Formel VII bzw. der Formel VIII nicht durch Substituenten im Benzolring aktiviert ist, im allgemeinen angebracht, zunächst in Gegenwart eines sauren Katalysators die Verbindungen der Formeln VII und III zum Hydrazon der Formel VIII zu kondensieren und die Cyclisierung dann in einem zweiten Schritt vorzunehmen, in dem man in Gegenwart einer Base arbeitet, durch die die Nucleophilie des β-Stickstoffatoms in der Hydrazongruppierung erhöht wird. Wird die Reaktion in zwei Stufen durchgeführt, so kann für die Cyclisierung des Hydrazons der Formel VIII dieses in Substanz isoliert werden oder es kann ohne Isolierung des Hydrazons das Reaktionsgemisch der Hydrazonherstellung in die Cyclisierung eingesetzt werden.

Die Umsetzungen von Verbindungen der Formel VII mit den Hydrazinen der Formel III oder deren Salzen

werden bevorzugt in einem Lösungsmittel oder Dispergiermittel vorgenommen. Geeignete Lösungsmittel sind zum Beispiel Wasser, Alkohole, Ether, Monoether und Diether des Ethylenglykols und des Di- und TriethylenglykolsEster, Amide, Nitrile, Säuren, Sulfoxide und Sulfone, Kohlenwasserstoffe und chlorierte Kohlenwasserstoffe. Beispiele für diese Lösungsmittel sind Methanol, Ethanol, n-Propanol, Isopropanol oder Butanole, Diethylether, Dipropylether, Dibutylether, Methyl-tert-butylether, Tetrahydrofuran oder Dioxan, Ethylenglykolmonomethylether, Ethylenglykolmonoethylether, Ethylenglykoldimethylether, Ethylenglykolmonobutylether, Diethylenglykolmonomethylether oder Diethylenglykoldimethylether, Essigsäureethylester oder Essigsäurebutylester, Dimethylformamid, Dimethylacetamid, N-Methylpyrrolidon oder Hexamethylphosphorsäuretriamid, Acetonitril, Essigsäure, Dimethylsulfoxid oder Sulfolan, Benzinfraktionen, Benzol, Toluol, Xylol, Chlorbenzol, Dichlorbenzol, Methylenchlorid oder Chloroform. Es können auch Mischungen von zwei oder mehr Lösungsmitteln eingesetzt werden. Bevorzugte Lösungsmittel sind Alkohole wie Methanol und Ethanol. Die Umsetzung wird im allgemeinen bei Temperaturen von 0 °C bis 150 °C, bevorzugt bei Temperaturen von 20 °C bis 130 °C durchgeführt. Besonders bevorzugt ist, sie unter Rückfluß bei der Siedetemperatur des verwendeten Lösungsmittels durchzuführen, zum Beispiel bei der Siedetemperatur des Methanols oder Ethanols.

Die Reaktionsdauer richtet sich nach dem Einzelfall und hängt zum Beispiel von der Reaktivität der Reaktionspartner und den Reaktionsbedingungen ab. Die Aufarbeitung des Reaktionsgemisches kann nach Standardverfahren erfolgen und das Produkt gewünschtenfalls nach üblichen Reinigungsmethoden, zum Beispiel durch Umkristallisation oder Chromatographie, gereinigt werden.

Werden freie Hydrazine der Formel III eingesetzt, so ist es vielfach besonders vorteilhaft, die Umsetzung mit den Verbindungen der Formel VII unter saurer Katalyse durchzuführen. Als Katalysatoren kommen zum Beispiel in Betracht organische Carbonsäuren und Sulfonsäuren wie Essigsäure, Trifluoressigsäure, Methansulfonsäure oder p-Toluolsulfonsäure, anorganische Säuren wie Chlorwasserstoff, Schwefelsäure oder Phosphorsäure, saure Salze wie Ammoniumsalze oder Hydrogenphosphate, oder saure Ionenaustauscher. Es kann auch günstig sein, einen bestimmten pH-Wert einzustellen oder in Gegenwart eines Puffersystems zu arbeiten. Die Art und Menge eines zugesetzten sauren Katalysators richten sich nach dem Einzelfall und hängen zum Beispiel von der Reaktivität der Reaktionspartner, dem Lösungsmittel oder der vorgesehenen Temperatur ab. Wird zum Beispiel eine Säure wie Essigsäure verwendet, so kann diese je nach der eingesetzten Menge sowohl als Lösungsmittel als auch als Katalysator fungieren. Bevorzugt ist es, die Umsetzung von Verbindungen der Formeln VII mit freien Hydrazinen der Formel III in Gegenwart von Essigsäure durchzuführen, Wird an Stelle eines freien Hydrazins ein Säureadditionssalz eines Hydrazins eingesetzt, zum Beispiel ein $R^{4'}$-$(CH_2)_n$-substituiertes Hydraziniumchlorid oder Hydraziniumsulfat, so wird damit bereits eine saure Verbindung in das Reaktionsgemisch eingebracht, die katalytisch wirken kann, und es ist vielfach vorteilhaft, zur Einstellung eines günstigen pH-Wertes einen Teil der eingebrachten Säure durch Zusatz einer gewissen Menge einer Base abzufangen, zum Beispiel durch Zusatz von Natriumacetat.

Das Mengenverhältnis, in dem die Verbindungen der Formeln III und VII vorteilhafterweise in die Reaktion eingesetzt werden, hängt vom Einzelfall ab. Es kann ungefähr 1:1 betragen, es kann aber auch ein Reaktionspartner in einem kleinerem oder in einem größerem Überschuß eingesetzt werden. Wenn zum Beispiel ein Reaktionspartner aufwendig in einer mehrstufigen Synthese hergestellt wird und der andere Reaktionspartner einfach zugänglich ist, kann es zur weitgehenden Ausnutzung des ersteren günstig sein, den letzteren im Überschuß einzusetzen, zum Beispiel in 1.1- bis 5-facher molarer Menge.

Wie bereits erwähnt, kann die Umsetzung der Verbindungen der Formeln VII und III auf der Stufe des Hydrazons der Formel VIII unterbrochen werden. Die dann in einem zweiten Schritt erfolgende Cyclisierung des Hydrazons zum Indazol der Formel Ia', die eine nucleophile Substitution der Gruppe $Z^1$ am Aromaten durch das β-Stickstoffatom der Hydrazonogruppe darstellt, kann je nach den im Einzelfall gegebenen Reaktivitäten zum Beispiel durch Erhitzen in einem Lösungsmittel oder Dispergiermittel erfolgen. In vielen Fällen ist es vorteilhaft, dabei eine Base zuzusetzen. Wenn bei der Herstellung von Verbindungen der Formel Ia' die Cyclisierung in einem separaten Schritt erfolgt, ist es bevorzugt, in diesem Schritt in Gegenwart einer Base zu arbeiten. Als Basen kommen zum Beispiel Hydroxide, Carbonate, Hydrogencarbonate, Hydride, Amide, Alkoholate oder metallorganische Verbindungen von Alkalimetallen wie Lithium, Natrium, Kalium oder Cesium oder Erdalkalimetallen wie Magnesium oder Calcium in Betracht. Bevorzugte Basen sind Alkalimetallalkoholate von ($C_1$-$C_4$)-Alkanolen wie Natrium- und Kaliummethylat, Natrium- und Kaliumethylat oder Natrium- und Kalium-tert-butylat. Es können auch Gemische von zwei oder mehr Basen eingesetzt werden. Die Base wird bevorzugt in äquimolarer Menge oder im Überschuß eingesetzt, üblicherweise in 1- bis 3- facher molarer Menge.

Als Lösungsmittel oder Dispergiermittel für eine in einem separaten Schritt durchgeführte Cyclisierung eines Hydrazons der Formel VIII zum Indazol kommen zum Beispiel Wasser, Alkohole, Ether, Monoether und Diether des Ethylenglykols und des Di- und Triethylenglykols, Ester, Amide, Nitrile, Sulfoxide, Sulfone, Kohlenwasserstoffe und chlorierte Kohlenwasserstoffe in Betracht. Beispiele für diese Lösungsmittel sind oben genannt. Es können auch Mischungen von zwei oder mehr Lösungsmitteln eingesetzt werden. Bevorzugte Lösungsmittel für eine Cyclisierung in Gegenwart einer Base sind aprotische Lösungsmittel, insbesondere dipolar aprotische Lösungsmittel wie Dimethylformamid, Dimethylacetamid, Hexamethylphosphorsäuretriamid, N-Methylpyrrolidon oder Dimethylsulfoxid.

Eine in einem separaten Schritt erfolgende Cyclisierung des Hydrazons der Formel VIII wird im allgemeinen

bei Temperaturen von 0 °C bis 150 °C, bevorzugt bei Temperaturen von 20 °C bis 130 °C durchgeführt. Besonders bevorzugt ist es wiederum, sie unter Rückfluß bei der Siedetemperatur des verwendeten Lösungsmittels oder Lösungsmittelgemisches durchzuführen. Die Aufarbeitung kann nach Standardmethoden erfolgen.

Ein weiteres Verfahren zur Herstellung von Verbindungen der Formel Ia bzw. Ia' neben der Umsetzung von Verbindungen der Formel VII mit $R^{4'}$-$(CH_2)_n$-substituierten Hydrazinen der Formel **III** ist die Umsetzung von Verbindungen der Formel VII mit Acylhydrazinen der Formel IX. In der Formel IX kann R beispielsweise für einen Alkylrest stehen, zum Beispiel für einen ($C_1$-$C_4$)-Alkylrest wie den Methylrest oder den tert-Butylrest, oder für einen Arylrest, zum Beispiel einen Phenylrest, der unsubstituiert oder substituiert sein kann. Ein Beispiel für eine geeignete Verbindung der Formel IX ist das Benzhydrazid. Die Umsetzung der Verbindungen der Formeln VII und IX kann unter den gleichen Bedingungen erfolgen wie oben für Umsetzung der Verbindungen der Formeln VII und III angegeben. Alle obigen Erläuterungen gelten hier entsprechend. Die Umsetzung der Verbindungen der Formeln VII und IX, die wiederum in einem Schritt oder in zwei Schritten erfolgen kann, führt zunächst zu den den Formeln VIII bzw. Ia'entsprechenden Acylhydrazonen bzw. Acylindazolen, die an Stelle der Gruppe $R^{4'}$-$(CH_2)_n$ die Gruppe R-CO enthalten. Nach Abspaltung der Acylgruppe werden aus diesen Verbindungen die Indazole der Formel X erhalten, in der X, $R^{1'}$, $R^{1a}$, $R^{5'}$

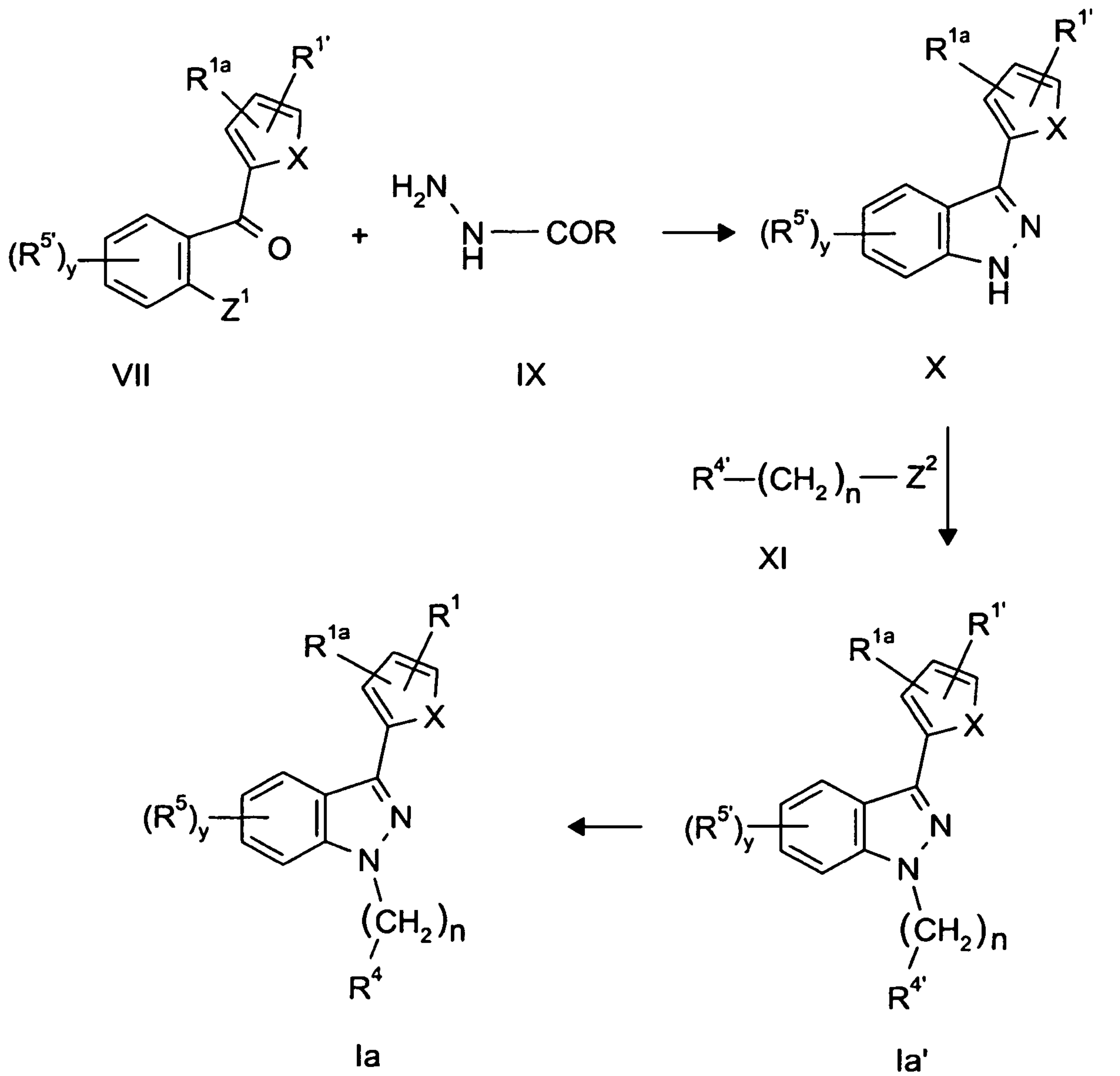

und y die oben für die Formeln Ia', VII und VIII angegebenen Bedeutungen haben. Für die Abspaltung der Acylgruppe können die Acylverbindungen zunächst isoliert werden, die Abspaltung kann aber auch ohne deren Isolierung in situ

erfolgen. Die Abspaltung der Acylgruppe läßt sich zum Beispiel in üblicher Weise durch Hydrolyse unter sauren oder basischen Bedingungen durchführen, zum Beispiel mittels Salzsäure, Schwefelsäure, Phosphorsäure, Lithiumhydroxid, Natriumhydroxid, Natriumcarbonat oder Kaliumhydroxid. Sie kann in Wasser oder in einem wasserhaltigen organischen Lösungsmittel oder Dispergiermittel erfolgen. Die Reaktionstemperatur und Reaktionsdauer richten sich nach dem Einzelfall, im allgemeinen wird bei Raumtemperatur bis 100 °C gearbeitet. Die in der 1-Position unsubstituierten Indazole der Formel X können nach den üblichen Methoden isoliert werden oder auch direkt in eine Folgereaktion eingesetzt werden. Die Indazole der Formel X können dann durch Umsetzung mit Alkylierungsmitteln der Formel XI in die 1-substituierten Indazole der Formel Ia' überführt werden. In der Formel XI haben $R^{4'}$ und n die oben angegebenen Bedeutungen, $Z^2$ steht für eine Abgangsgruppe, beispielsweise für Chlor, Brom, Iod oder einen Sulfonyloxyrest wie Methansulfonyloxy, Trifluormethansulfonyloxy, Benzolsulfonyloxy oder p-Toluolsulfonyloxy. Im allgemeinen wird das Alkylierungsmittel der Formel XI in äquimolarer Menge oder im Überschuß eingesetzt, zum Beispiel in 1- bis 3-facher molarer Menge.

Die Alkylierung der Verbindungen der Formel X kann unter den üblichen Alkylierungsbedingungen erfolgen. Bevorzugt wird sie in einem Lösungsmittel oder Dispergiermittel durchgeführt, zum Beispiel in Wasser, einem Alkohol, einem Ether, einem Monoether oder Diether des Ethylenglykols oder des Di- und Triethylenglykols, einem Keton wie Aceton oder Methylethylketon, einem Ester, einem Amid, einem Nitril, einem Sulfoxid oder Sulfon, einem Kohlenwasserstoff oder einem chlorierten Kohlenwasserstoff. Die oben angegebenen Beispiele für diese Lösungsmittel gelten auch hier. Es können auch Mischungen von zwei oder mehr Lösungsmitteln eingesetzt werden, beispielsweise Mischungen eines organischen Lösungsmittels mit Wasser. Bevorzugt wird die Alkylierung in Wasser oder in einem dipolar aprotischen Lösungsmittel, zum Beispiel Dimethylformamid, durchgeführt. Die Alkylierung wird im allgemeinen bei Temperaturen von 0 °C bis 150 °C, bevorzugt bei Temperaturen von 20 °C bis 130 °C durchgeführt. Besonders bevorzugt ist, sie unter Rückfluß bei der Siedetemperatur des verwendeten Lösungsmittels durchzuführen.

Die Alkylierung der Verbindungen der Formel X mit den Verbindungen der Formel XI erfolgt bevorzugt unter Zusatz einer Base. Geeignete Basen sind zum Beispiel die Hydroxide, Carbonate, Acetate, Hydride oder Alkoholate von Alkalimetallen wie Lithium, Natrium oder Kalium oder von Erdalkalimetallen wie Magnesium oder Calcium. Es können auch Gemische von Basen verwendet werden. Im allgemeinen wird die Base in äquimolarer Menge oder im Überschuß eingesetzt, zum Beispiel in 1- bis 3-facher molarer Menge. Besonders bevorzugt sind die Alkylierung unter Verwendung von Kalium-tert-butylat oder Natriumhydrid in Dimethylformamid und die Verwendung von Natriumhydroxid in Wasser. Die Aufarbeitung des Reaktionsgemisches kann nach Standardverfahren erfolgen und das Produkt gewünschtenfalls nach üblichen Reinigungsmethoden, zum Beispiel durch Umkristallisation oder Chromatographie, gereinigt werden.

In den Verbindungen der Formel Ia' können, wie schon oben gesagt, die Reste $R^{1'}$, $R^{4'}$ und $R^{5'}$ sowie y die in den Definition von $R^1$, $R^4$ und $R^5$ angegebenen Bedeutungen haben, so daß die nach den erläuterten Syntheseverfahren erhaltenen Reaktionsprodukte der Formel Ia' bereits erfindungsgemäße Verbindungen der Formel Ia darstellen. Es können aber in den nach den erläuterten Syntheseverfahren erhaltenen Verbindungen der Formel Ia' auch noch in vielfältiger Weise strukturelle Abwandlungen vorgenommen werden. Wie schon gesagt, kann es sich dabei um die Freisetzung von funktionellen Gruppen handeln, die während der Synthese in geschützter Form vorlagen. Es können aber auch in erfindungsgemäße Verbindungen der Formel Ia' nach üblichen chemischen Methoden zusätzliche funktionelle Gruppen eingeführt werden oder in erfindungsgemäßen Verbindungen vorhandene Strukturelemente oder funktionelle Gruppe nach üblichen Methoden abgewandelt werden. Diese Methoden sind dem Fachmann bekannt und in Standardwerken ausführlich beschrieben, zum Beispiel in Houben-Weyl, Methoden der Organischen Chemie, Thieme-Verlag, Stuttgart, oder Organic Reactions, John Wiley & Sons, New York. Eine gegebenenfalls notwendige Anpassung von Reaktionsbedingungen an die Reaktivität der Verbindungen der Formel Ia' bereitet dem Fachmann keine Probleme. Beispielhaft seien als in Betracht kommende Reaktionstypen genannt:

Hydrolyse von Carbonsäureestern zu den Carbonsäuren bzw. den Alkoholen

Überführung von Carbonsäuren in die Carbonsäureester durch Veresterung mit Alkoholen in Gegenwart von Säure, Überführung von Carbonsäuren in in Carbonsäureester durch in situ-Aktivierung der Carbonsäuren und Umsetzung mit Alkoholen oder Überführung von Carbonsäuren in reaktive Derivate, zum Beispiel in die Carbonsäurechloride durch Umsetzung der Säuren oder ihrer Salze mit Chlorierungsmitteln wie Thionylchlorid, Oxalylchlorid oder Phosphorhalogeniden, und Umsetzung der reaktiven Derivate mit Alkoholen zu Carbonsäureestern.

Reduktion von Carbonsäurederivaten zu Aldehyden oder Alkoholen und Reduktion von Aldehyden und Ketonen zu Alkoholen sowie Addition von metallorganischen Verbindungen wie Grignard-Verbindungen oder lithiumorganischen Verbindungen an Carbonsäuregruppen oder Aldehyd- oder Ketongruppen unter Bildung von Ketonen oder Alkoholen

Oxidation von Alkoholen zu Aldehyden oder Ketonen

Veretherung, Halogenierung und Veresterung von Alkoholen

Nucleophile Substitutionen an aliphatischen Kohlenstoffatomen.

Elektrophile aromatische Substitution unter Ersatz eines Wasserstoffatoms an einem carbocyclischen oder heterocylischen aromatischen Ring durch eine funktionelle Gruppe, zum Beispiel Halogenierung.

Die Ausgangsprodukte der Formel VII sind bekannt oder können analog zu bekannten Verbindungen nach wohlbekannten, in der Literatur beschriebenen Standardverfahren hergestellt werden. Die Aromaten der Formel VII können zum Beispiel durch Friedel-Crafts-Acylierungen von Furanen mit Benzoesäurederivaten wie zum Beispiel Säurechloriden erhalten werden. Näheres zu diesen Reaktionen findet sich in den Standardwerken wie Houben-Weyl oder Organic Reactions (siehe oben). Auch die Hydrazine der Formeln III und IX sowie die Alkylierungsmittel der Formel XI sind bekannt oder können nach wohlbekannten Verfahren hergestellt werden, zu denen sich in in diesen Standardwerken nähere Angaben finden.

Die erfindungsgemäßen Verbindungen der Formel I bewirken über die Aktivierung der löslichen Guanylat-Cyclase (sGC) eine Erhöhung der cGMP-Konzentration und sind deshalb wertvolle Agenzien zur Therapie und Prophylaxe von Krankheiten, die mit einem niedrigen oder erniedrigten cGMP-Spiegel verbunden sind oder durch einen solchen verursacht werden oder zu deren Therapie oder Prophylaxe eine Erhöhung des vorhandenen cGMP-Spiegels angestrebt wird. Die Aktivierung der sGC durch die Verbindungen der Formel I kann zum Beispiel in dem unten beschriebenen Aktivitätsassay untersucht werden, ihre Organwirkung zum Beispiel durch die Bestimmung der Relaxation der Ratten-Aorta.

Krankheiten und pathologische Zustände, die mit einem niedrigen cGMP-Spiegel verbunden sind oder bei denen eine Erhöhung des cGMP-Spiegels angestrebt wird und zu deren Therapie und Prophylaxe Verbindungen der Formel I eingesetzt werden können, sind zum Beispiel Herz-Kreislauf-Erkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, oder zum Beispiel erektile Dysfunktion, Asthma bronchiale, chronische Niereninsuffizienz und Diabetes. Verbindungen der Formel I können darüber hinaus eingesetzt werden bei der Therapie der Leberzirrhose sowie aufgrund ihrer zum Teil synergistischen Wirkung mit der retrograden Messenger-Substanz NO zur Verbesserung einer eingeschränkten Lernfähigkeit oder Gedächtnisleistung.

Die Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind daher auch die Verbindungen der Formel I und ihre physiologisch verträglichen Salze zur Verwendung als Arzneimittel, ihre Verwendung zur Normalisierung eines gestörten cGMP-Haushalts und insbesondere ihre Verwendung in der Therapie und Prophylaxe der oben genannten Krankheitsbilder, sowie ihre Verwendung zur Herstellung von Medikamenten dafür. Weiterhin sind Gegenstand der vorliegenden Erfindung pharmazeutische Präparate, die als aktiven Bestandteil eine wirksame Dosis mindestens einer Verbindung der Formel I und/oder eines physiologisch verträglichen Salzes davon neben üblichen pharmazeutisch einwandfreien Trägerstoffen und Zusatzstoffen enthalten.

Die Arzneimittel können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen, oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die pharmazeutischen Präparate enthalten normalerweise 0.5 bis 90 Gewichtsprozent der Verbindungen der Formel I und/oder ihrer physiologisch verträglichen Salze. Die Herstellung der pharmazeutischen Präparate kann in an sich bekannter Weise erfolgen. Dazu werden ein oder mehrere Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen mit therapeutischer oder prophylaktischer Wirkung in eine geeignete Verabreichungsform bzw. Dosierungsform gebracht, die dann als Arzneimittel in der Humanmedizin oder Veterinärmedizin verwendet werden kann.

Für die Herstellung beispielsweise von Pillen, Tabletten, Dragees und Hartgelatinekapsein kann man Lactose, Stärke, zum Beispiel Maisstärke, oder Stärkederivate, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle etc. Die Verbindungen der Formel I und ihre physiologisch verträglichen Salze können auch lyophilisiert werden und die erhaltenen Lyophilisate zum Beispiel zur Herstellung von Injektions- oder Infusionspräparaten verwendet werden. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch übliche Zusatzstoffe

enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, Salze zur Veränderung des osmotischen Drucks, Überzugsmittel oder Antioxidantien.

Die Dosierung des zu verabreichenden Wirkstoffs der Formel I und/oder eines physiologisch verträglichen Salze davon hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel I weitere Wirkstoffe verabreicht werden. Im allgemeinen ist eine Tagesdosis von etwa 0.01 bis 100 mg/kg, vorzugsweise 0.1 bis 10 mg/kg, insbesondere 0.3 bis 5 mg/kg (jeweils mg pro kg Körpergewicht) bei Verabreichung an einen ca. 75 kg schweren Erwachsenen zur Erzielung wirksamer Ergebnisse angemessen. Die Tagesdosis kann in einer Einzeldosis verabreicht werden oder, insbesondere bei der Applikation größerer Mengen, in mehrere, zum Beispiel zwei, drei oder vier Einzeldosen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0.2 bis 500 mg, vorzugsweise 1 bis 200 mg Wirkstoff der Formel I und/oder dessen physiologisch verträgliche Salze pro Dosis.

Die Verbindungen der Formel I aktivieren die lösliche Guanylatcyclase. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Beeinflussung der Guanylatcyclase beabsichtigt ist, sowie für diagnostische Zwecke, zum Beispiel in der in vitro-Diagnostik von Zell- oder Gewebsproben. Ferner können die Verbindungen der Formel I und ihre Salze, wie bereits oben erwähnt, als Zwischenprodukte zur Herstellung weiterer Arzneimittelwirkstoffe dienen.

Die nachfolgenden Beispiele erläutern die Erfindung, ohne sie einzuschränken.

In den Versuchsbeschreibungen bedeuten:

| | |
|---|---|
| RT | Raumtemperatur |
| THF | Tetrahydrofuran |
| DMF | Dimethylformamid |

Beispiel 1

1-Benzyl-3-(5-carboxy-2-furyl)-indazol-Kaliumsalz (Ausgangsmaterial)

1a) 5-(2-Fluor-benzoyl)-furan-2-carbonsäure

132.6 g (0.53 mol) 5-(2-Fluor-benzoyl)-furan-2-carbonsäuremethylester wurden in eine Lösung aus 31.47 g (0.56 mol) Kaliumhydroxid in 350 ml Wasser eingetragen und auf 70 °C erwärmt. Nach 1 h wurde die Lösung filtriert und mit konz. Salzsäure sauer gestellt. Der Niederschlag wurde abgesaugt und aus Ethanol umkristallisiert. Man erhielt 106.3 g (85 %) der Titelverbindung. Schmp.: 195-196 °C

1b) 1-Benzyl-3-(5-carboxy-2-furyl)-indazol-Kaliumsalz

70.26 g (0.3 mol) 5-(2-Fluor-benzoyl)-furan-2-carbonsäure wurden in 350 ml Methanol vorgelegt, 109.95 g (0.9 mol) Benzylhydrazin wurden zugesetzt und nach Zugabe von 8 ml Eisessig wurde 6 h unter Rückfluß erhitzt. Zur Aufarbeitung wurde einrotiert, der Rückstand in 2 N Natronlauge aufgenommen und mit Essigester extrahiert. Die wäßrige Phase wurde mit 2 N Salzsäure sauer gestellt und mit Essigester extrahiert. Der nach Trocknen und Einrotieren verbliebene Rückstand wurde aus Essigester/n-Hexan umkristallisiert. Die auf diese Weise erhaltenen 68.3 g (0.20 mol) 5-(2-Fluor-benzoyl)-furan-2-carbonsäure-Benzylhydrazon (Schmp.: 147 °C (Zers.)) wurden in 400 ml DMF gelöst, 45.38 g (0.40 mol) Kalium-tert-butylat wurden eingetragen und es wurde 30 min unter Rückfluß erhitzt. Der ausgefallene Niederschlag wurde abgesaugt, mit Dichlormethan gewaschen und aus Ethanol/Wasser (95:5) umkristallisiert. Man erhielt 57.7 g (54 %) der Titelverbindung. Die entsprechende Cyclisierung der in der Mutterlauge noch enthaltenen Vorstufe ergab 25.9 g Produkt.
Schmp.: > 300 °C
$^1$H-NMR ($D_6$-DMSO): δ = 5.73 (s, 2H, $CH_2$), 6.68 (d; 1H, H-3'), 6.92 (d, 1H, H-4'), 7.18-7.38 (m, 6H, Phenyl-H, H-5), 7.45 (t, 1H, H-6), 7.75 (d, 1H, H-7), 8.18 (d, 1H, H-4)

Beispiel 2

1-Benzyl-3-(5-ethoxycarbonyl-2-furyl)-indazol (Ausgangsmaterial)

52.4 g (0.15 mol) 1-Benzyl-3-(5-carboxy-2-furyl)-indazol-Kaliumsalz wurden in 1250 ml Toluol vorgelegt und nach Zugabe von 200 ml abs. Ethanol und 50 ml konz. Schwefelsäure am Wasserabscheider unter Rückfluß gerührt. Nach 3 h wurde einrotiert, das verbliebene Öl in Wasser/Essigester aufgenommen und die Wasserphase abgetrennt. Die organische Phase wurde mit Wasser und 7.5 %iger $NaHCO_3$-Lösung gewaschen, mit Natriumsulfat getrocknet und eingeengt. Der Rückstand wurde aus Isopropanol umkristallisiert. Man erhielt 37.8 g (73 %) der Titelverbindung. Schmp.: 98-99 °C

Beispiel 3

1-Benzyl-3-(5-hydroxymethyl-2-furyl)-indazol (Ausgangsmaterial)

2.06 g (54.6 mmol) Lithiumaluminiumhydrid wurden in 250 ml absolutem THF vorgelegt und eine Lösung von 18.8 g (54.6 mmol) 1-Benzyl-3-(5-ethoxycarbonyl-2-furyl)-indazol in 250 ml THF wurde zugetropft. Nach 45 min wurde mit 25 %iger Kaliumcarbonatlösung versetzt, 30 min bei RT nachgerührt, abgesaugt und der Niederschlag mit THF gewaschen. Die vereinigten organischen Phasen wurden einrotiert und der Rückstand wurde aus Isopropanol umkristallisiert. Man erhielt 11.0 g (67 %) der Titelverbindung.
Schmp.: 113-114 °C

Entsprechend wurden hergestellt:

Beispiel 4 (Ausgangsmaterial) 3-(5-Carboxy-2-furyl)-1-(3,5-bis(trifluormethyl)-phenyl)-indazol

Schmp.: 256-257 °C

Beispiel 5 (Ausgangsmaterial) 3-(5-Ethoxycarbonyl-2-furyl)-1-(3,5-bis(trifluormethyl)-phenyl)-indazol

Schmp.: 128-129 °C

Beispiel 6 (Ausgangsmaterial) 3-(5-Hydroxymethyl-2-furyl)-1-(3,5-bis(trifluormethyl)-phenyl)-indazol

Schmp.: 136-138°C

Beispiel 8 (Ausgangsmaterial) 1-Benzyl-3-(5-methoxycarbonyl-2-furyl)-5-nitro-indazol

8a) 5-(2-Fluor-5-nitro-benzoyl)-furan-2-carbonsäuremethylester

Zu einer Suspension von 2 g Eisen-(III)-chlorid und 29.0 g (0.14 mol) 2-Fluor-5-nitro-benzoylchlorid in 100 ml getrocknetem Tetrachlorkohlenstoff wurde eine Lösung von 24.7 g (0.2 mol) Furan-2-carbonsäuremethylester in 50 ml getrocknetem Tetrachlorkohlenstoff zugetropft und 14 h unter Rückfluß (80 °C) erhitzt.

Anschließend wurden 50 ml Methanol zugesetzt, es wurde 30 min bei RT gerührt und einrotiert. Der verbliebene Rückstand wurde in Essigester aufgenommen, mit Wasser und $NaHCO_3$-Lösung gewaschen, mit Natriumsulfat getrocknet, einrotiert und aus Isopropanol umkristallisiert. Man erhielt 3.5 g (9 %) der Titelverbindung. Schmp.: 134-135 °C

8b) 1-Benzyl-3-(5-methoxycarbonyl-2-furyl)-5-nitro-indazol

2.6 g (9 mmol) 5-(2-Fluor-5-nitro-benzoyl)-furan-2-carbonsäuremethylester und 3.31g (27 mmol) Benzylhydrazin wurden in ca. 60 ml Methanol vorgelegt und nach Zugabe von 0.2 ml Eisessig 15 min zum Rückfluß erwärmt. Der ausgefallene Niederschlag wurde abgesaugt, mit wenig Methanol gewaschen und im Vakuumtrockenschrank bei RT getrocknet. Das Rohprodukt wurde durch Chromatographie an Kieselgel mit Dichlormethan gereinigt. Man erhielt 2.9 g (85 %) der Titelverbindung.
Schmp.: 171-173 °C

Beispiel 9 (Ausgangsmaterial) 5-Amino-1-benzyl-3-(5-methoxycarbonyl-2-furyl)-indazol

0.9 g (2.4 mmol) 1-Benzyl-3-(5-methoxycarbonyl-2-furyl)-5-nitro-indazol wurden durch leichtes Erwärmen in

100 ml Methanol/THF (1:1) gelöst, eine Lösung von 2.5 g (14.4 mmol) Natriumdithionit in 50 ml Wasser wurde zugegeben und das Gemisch wurde 16 h bei RT gerührt. Zur Aufarbeitung wurde eingeengt und der Rückstand an Kieselgel mit Dichlormethan/Methanol (98.5:1.5) chromatographiert. Man erhielt 140 mg (17 %) der Titelverbindung.
Schmp.: 195-196 °C

Beispiel 10 (Ausgangsmaterial) 5-Amino-1-benzyl-3-(5-hydroxymethyl-2-furyl)-indazol

Zu 0.01 g (0.3 mmol) Lithiumaluminiumhydrid in 5 ml THF wurde eine Lösung von 80 mg (0.23 mmol) 5-Amino-1-benzyl-3-(5-methoxycarbonyl-2-furyl)-indazol in 5 ml THF getropft und bei RT gerührt. Nach 3 h wurden 10 ml 25 %ige $K_2CO_3$-Lösung zugegeben und es wurde 30 min nachgerührt. Der Niederschlag wurde abgesaugt, mit THF ausgekocht, die vereinigten organischen Phasen wurden getrocknet, eingeengt und der Rückstand an Kieselgel mit Dichlormethan/Methanol (95:5) chromatographiert. Man erhielt 21 mg (29 %) der Titelverbindung.
Schmp.: 156 °C

Beispiel 11 (Ausgangsmaterial) 1-Benzyl-3-(5-carboxy-2-furyl)-6-nitro-indazol

11 a) 5-(2-Fluor-4-nitro-benzoyl)-furan-2-carbonsäuremethylester

8.4 g Eisen-(III)-chlorid, 44.8 g (0.22 mol) 2-Fluor-4-nitro-benzoylchlorid und 33.4 g (0.26 mol) Furan-2-carbonsäuremethylester wurden in 80 ml Tetrachlorkohlenstoff 2 Tage unter Rückfluß erhitzt. Zur Aufarbeitung wurden 100 ml Methanol zugefügt, es wurde 10 min gerührt, eingeengt, der Rückstand in Essigester/Wasser aufgenommen und die organische Phase mehrfach mit $Na_2CO_3$-Lösung ausgeschüttelt. Der nach dem Trocknen und Einengen der Essigester-Phase verbliebene Rückstand wurde mit Methanol ausgezogen und der unlösliche Anteil an Kieselgel mit Dichlormethan chromatographiert. Man erhielt 19.0 g (29 %) der Titelverbindung.
Schmp.: 136-138 °C

11b) 5-(2-Fluor-4-nitro-benzoyl)-furan-2-carbonsäure

14.5 g (49 mmol) 5-(2-Fluor-4-nitro-benzoyl)-furan-2-carbonsäuremethylester wurden zu 200 ml 0.1 N Natronlauge gegeben und 3 Tage bei RT gerührt. Anschließend wurde mit 1 N Salzsäure auf pH 4 gestellt, 30 min im Eisbad gekühlt und abgesaugt. Man erhielt 8.6 g (62 %) der Titelverbindung.
Schmp.: 170 °C (Zers.)
$^1$H-NMR ($D_6$-DMSO): $\delta$ = 7.39 (d, 1H, H-4'), 7.50 (d, 1H, H-3'), 8.00 (dd, 1H, H-6), 8.25 (dd, 1H, H-5), 8.34 (dd, 1H, H-3)

11c) 1-Benzyl-3-(5-carboxy-2-furyl)-6-nitro-indazol

8.5 g (30 mmol) 5-(2-Fluor-4-nitro-benzoyl)-furan-2-carbonsäure wurden in 100 ml Methanol gelöst, mit 11.2 g (91 mmol) Benzylhydrazin versetzt und 7 h unter Rückfluß erhitzt. Anschließend wurde in Wasser gegossen, mit konz. Salzsäure auf pH 4 gestellt und mit Essigester extrahiert. Die vereinigten organischen Phasen wurden getrocknet und eingeengt. Das rohe Zwischenprodukt wurde zur Cyclisierung in 50 ml DMF gelöst, 6.8 g (61 mmol) Kalium-tert-butylat wurden zugegeben und es wurde 3 h unter Rückfluß erhitzt. Zur Aufarbeitung wurde eingeengt, mit 1 N Salzsäure auf pH 4 gestellt, mit Essigester extrahiert, die organische Phase eingeengt und der Rückstand an Kieselgel mit Essigester/Eisessig (60:1) chromatographiert. Man erhielt 8 g (ca. 73 %) der Titelverbindung als Öl.
$^1$H-NMR ($D_6$-DMSO): $\delta$ = 5.96 (s, 2H, $CH_2$), 7.22-7.41 (m, 7H, Phenyl-H, H-3', H-4'), 8.13 (dd, 1H, H-5), 8.38 (d, 1H, H-4), 8.93 (d, 1H, H-7)

Beispiel 12 (Ausgangsmaterial) 3-(5-Carboxy-2-furyl)-1-(2-phenylethyl)-indazol

5 g (21 mmol) 5-(2-Fluorbenzoyl)-furan-2-carbonsäure, 12 g (51 mmol) (2-Phenylethyl)-hydrazinium-Sulfat und 8.4 g (102 mmol) Natriumacetat wurden in 50 ml Ethanol 12 h unter Rückfluß gekocht. Anschließend wurde eingeengt, mit Wasser/Essigester verrührt, die Essigesterphase abgetrennt, getrocknet und eingeengt. Das rohe Zwischenprodukt (Hydrazon) wurde zur Reinigung an Kieselgel mit Dichlormethan/Methanol (9:1) chromatographiert. 4 g (11.3 mmol) des Hydrazons wurden in 20 ml DMF gelöst und mit 2.5 g (23 mmol) Kalium-tert-butylat 3 h unter Rückfluß erhitzt. Das ausgefallene Kaliumsalz der Titelsäure wurde abgesaugt. Das eingeengte Filtrat wurde an Kieselgel mit Dichlormethan/Methanol (7:3) chromatographiert. Man erhielt insgesamt 1.9 g (27 %, berechnet für die Säure) der Titelverbindung. Als Nebenprodukt bildete sich 3-(5-Carboxy-2-furyl)-indazol.
Schmp.: Zers. > 190 °C
$^1$H-NMR ($D_6$-DMSO): $\delta$ = 3.20 (t, 2H, $C\underline{H_2}$-Phenyl), 4.65 (t, 2H, $CH_2$-N), 7.00 (m, 2H, H-3', H-4'), 7.13-7.23 (m, 6H,

Phenyl-H, H-5), 7.38 (t, 1H, H-6), 7.60 (d, 1H, H-7), 8.14 (d, 1H, H-4)

Beispiel 13 (Ausgangsmaterial) 3-(5-Ethoxycarbonyl-2-furyl)-1-(2-phenylethyl)-indazol

0.8 g (2.4 mmol) 3-(5-Carboxy-2-furyl)-1-(2-phenylethyl)-indazol wurden mit 25 ml Ethanol, 150 ml Toluol und 2 ml konz. Schwefelsäure versetzt und 3 h am Wasserabscheider erhitzt. Chromatographie des nach Einrotieren erhaltenen Rückstandes an Kieselgel mit Dichlormethan/Hexan (2:1) ergab 400 mg (46 %) der Titelverbindung.
$^1$H-NMR ($D_6$-DMSO): δ = 1.40 (t, 3H, $CH_3$), 3.34 (t, 2H, $C\underline{H_2}$-Phenyl), 4.35 (q, 2H $OCH_2$), 4.73 (t, 2H, $CH_2$-N), 7.08-7.37 (m, 6H, Phenyl-H, H-3'), 7.38 (t, 1H, H-5), 7.45 (t, 1H, H-6), 7.48 (d, 1H, H-4'), 7.65 (d, 1H, H-7), 8.10 (d, 1H, H-4)

Beispiel 14 (Ausgangsmaterial) 3-(5-Hydroxymethyl-2-furyl)-1-(2-phenylethyl)-indazol

Zu 31.5 mg (0.83 mmol) Lithiumaluminiumhydrid in 10 ml THF wurde eine Lösung von 300 mg (0.83 mmol) 3-(5-Ethoxycarbonyl-2-furyl)-1-(2-phenylethyl)-indazol in 10 ml THF zugetropft. Nach 1 h wurde mit 25 %iger Kaliumcarbonat-Lösung versetzt, der Niederschlag abgetrennt und mit THF gewaschen. Die vereinigten THF-Filtrate wurden getrocknet und eingeengt. Das Rohprodukt wurde durch Chromatographie an Kieselgel mit Dichlormethan/Methanol (95:5) gereinigt. Man erhielt 200 mg (75 %) der Titelverbindung.
$^1$H-NMR ($D_6$-DMSO): δ = 3.20 (t, 2H, $CH_2$-Phenyl ), 4.53 (bs, 2H, $CH_2O$), 4.66 (t, 2H, $CH_2$-N), 6.50 (d, 1H, H-3'), 6.98 (d, 1H, H-4'), 7.15-7.27 (m, 6H, Phenyl-H, H-5), 7.38 (t, 1H, H-6), 7.58 (d, 1H, H-7), 8.08 (d, 1H, H-4)

Beispiel 15 (Ausgangsmaterial) 3-(5-Carboxy-2-furyl)-indazol

2.5 g (11 mmol) 5-(2-Fluorbenzoyl)-furan-2-carbonsäure, 3.28 g (24 mmol) Benzhydrazid und 2 Tropfen Eisessig wurden in 50 ml Ethanol 10 h unter Rückfluß erhitzt. Anschließend wurde einrotiert, der Rückstand mit Wasser aufgenommen, mit 2 N $NaHCO_3$-Lösung basisch gestellt und mit Essigester extrahiert. Die wäßrige Phase wurde mit 2 N Salzsäure sauer gestellt und mit Essigester extrahiert. Die vereinigten organischen Phasen wurden getrocknet und einrotiert. Der Rückstand wurde in 25 ml DMF gelöst, 2.74 g (24.2 mmol) Kalium-tert-butylat wurden zugesetzt und es wurde 1.5 h bei Rückflußtemperatur gerührt. Nach Abkühlen wurde einrotiert, der Rückstand in 1 N Natronlauge aufgenommen und mit Essigester extrahiert. Der beim Ansäuern der wäßrigen Phase ausgefallene Niederschlag wurde abgesaugt, mit Wasser gewaschen und im Vakuumtrockenschrank bei 40 °C getrocknet. Man erhielt 1.88 g (75 %) der Titelverbindung.
Schmp.: 205 °C (Zers.)
$^1$H-NMR ($D_6$-DMSO): δ = 7.15 (d, 1H, H-3'), 7,28 (t, 1H, H-5), 7.40 (d, 1H, H-4'), 7.48 (t, 1H, H-6), 7.63 (d, 1H, H-7), 8.15 (d, 1H, H-4), 13.1 (bs, 1H, COOH), 13.54 (bs, 1H, H-1)

Beispiel 16 (Ausgangsmaterial) 3-(5-Ethoxycarbonyl-2-furyl)-indazol

1.75 g (7.7 mmol) 3-(5-Carboxy-2-furyl)-indazol wurden zusammen mit 2.5 ml konzentrierter Schwefelsäure in 60 ml Toluol und 20 ml Ethanol am Wasserabscheider erhitzt. Übliche Aufarbeitung ergab 1.2 g (60 %) der Titelverbindung.
$^1$H-NMR ($D_6$-DMSO): δ = 1.36 (t, 3H, $CH_3$), 4.35 (q, 2H, $CH_2$), 7.10 (d, 1H, H-3'), 7.30 (t, 1H, H-5), 7.44 (t, 1H, H-6), 7.46 (d, 1H, H-4'), 7.64 (d, 1H, H-7), 8.13 (d, 1H, H-4), 13.60 (bs, 1H, H-1)

Beispiel 17 (Ausgangsmaterial) 1-((5-Chlor-2-thienyl)-methyl)-3-(5-ethoxycarbonyl-2-furyl)-indazol

600 mg (2.3 mmol) 3-(5-Ethoxycarbonyl-2-furyl)-indazol wurden zusammen mit 265 mg (2.4 mmol) Kalium-tert-butylat in 10 ml DMF vorgelegt, 430 mg (2.6 mmol) 2-Chlor-5-(chlormethyl)-thiophen wurden in 1 ml DMF zugetropft und es wurde 2 h bei RT gerührt. Übliche Aufarbeitung ergab 350 mg (41 %) der Titelverbindung.
Schmp.: 124-126 °C

Beispiel 18 (Ausgangsmaterial) 1-((5-Chlor-2-thienyl)-methyl)-3-(5-hydroxymethyl-2-furyl)-indazol

Die Verbindung wurde analog Beispiel 14 aus 1-((5-Chlor-2-thienyl)-methyl)-3-(5-ethoxycarbonyl-2-furyl)-indazol durch Reduktion mit Lithiumaluminiumhydrid hergestellt.
$^1$H-NMR ($D_6$-DMSO): δ = 4.50 (d, 2H, $CH_2O$), 5.35 (t, 1H, OH), 5.88 (s, 2H, N-$CH_2$), 6.48 (d, 1H, Thiophen-H-3), 6.98 (m, 2H, H-3', Thiophen-H-4), 7.26 (t, 1H, H-5), 7.41 (d, 1H, H-4'), 7.48 (t, 1H, H-6), 7.81 (d, 1H, H-7), 8.10 (d, 1H, H-4)

Beispiel 41 (Ausgangsmaterial) 1-Benryl-3-(5-formyl-2-furyl)-indazol

7.7 g (25.3 mmol) 1-Benzyl-3-(5-hydroxymethyl-2-furyl)-indazol und 11g (126.5 mmol) aktiviertes Mangan-(IV)-oxid wurden in 200 ml trockenem Tetrachlorkohlenstoff 4.5 h unter Rückfluß gerührt. Zur Aufarbeitung wurde abgesaugt, das Filtrat mit Wasser gewaschen, getrocknet und einrotiert. Das Rohprodukt wurde aus Isopropanol umkristallisiert. Man erhielt 4.8 g (63 %) der Titelverbindung.
Schmp.: 108 °C

Beispiel 42 (Bezugsbeispiel) 1-Benzyl-3-(5-(1-hydroxypropyl)-2-furyl)-indazol

Zu 300 mg (0.99 mmol) 1-Benzyl-3-(5-formyl-2-furyl)-indazol in 20 ml Diethylether wurden bei 10 °C 1.1 ml (1.1 mmol) einer 1 M Lösung von Ethylmagnesiumbromid in THF getropft. Nach 1 h bei 10 °C wurde auf Eiswasser gegossen, mit Essigester extrahiert, getrocknet und eingeengt. Das Rohprodukt wurde durch Chromatographie an Kieselgel mit Dichlormethan/Methanol (95:5) gereinigt. Man erhielt 200 mg (61 %) der Titelverbindung.
$^1$H-NMR ($D_6$-DMSO): $\delta$ = 0.95 (t, 3H, $CH_3$), 1.80 (m, 2H, $CH_2$-C-O), 4.55 (m, 1H, CH-O), 5.36 (bs, 1H, OH), 5.78 (s, 2H, $CH_2$-Phenyl), 6.43 (d, 1H, H-3'), 6.94 (d, 1H, H-4'), 7.20-7.38 (m, 6H, Phenyl-H, H-5), 7.45 (t, 1H, H-6), 7.75 (d, 1H, H-7), 8.12 (d, 1H, H-4)
Analog Beispiel 42 wurde erhalten:

Beispiel 44

1-Benzyl-5-(5-(1-hydroxy-prop-2-in-1-yl)-2-furyl)-indazol

Schmp.: 151 °C

Beispiel 50 (Ausgangsmaterial) 3-(5-Carboxy-2-furyl)-1-(4-trifluormethylpyrimidin-2-yl)-indazol-Hydrochlorid

1.3 g (5.7 mmol) 3-(5-Carboxy-2-furyl)-indazol wurden zusammen mit 1.3 g (11.4 mmol) Kalium-tert-butylat in 5 ml trockenem DMF gelöst und mit 2-Chlor-4-trifluormethyl-pyrimidin, gelöst in 5 ml DMF, versetzt. Nach 3 h Rühren bei 60 °C wurde eingeengt, in 500 ml Wasser aufgenommen, mit Essigester extrahiert und das Hydrochlorid aus der wäßrigen Phase durch Zugabe von 1 N Salzsäure abgeschieden. Man erhielt 420 mg (25 %) der Titelverbindung.
Schmp.: 258-261 °C

Pharmakologische Untersuchungen

1) Aktivierung der löslichen Guanylatcyclase

Die Aktivierung der löslichen Guanylatcyclase (sGC), die die Umwandlung von Guanosintriphosphat (GTP) in cyclisches Guanosinmonophosphat (cGMP) und Pyrophosphat katalysiert, durch die erfindungsgemäßen Verbindungen wurde mit Hilfe eines Enyzm-Immuno-Assays (EIA) der Firma Amersham quantifiziert. Dazu wurden die Prüfsubstanzen zunächst mit sGC in Mikrotiterplatten inkubiert und dann die Menge des entstandenen cGMP bestimmt.
Die eingesetzte sGC war aus Rinderlunge isoliert worden (siehe Methods in Enzymology, Band 195, S. 377). Die Testlösungen (100 $\mu$l pro well) enthielten 50 mM Triethanolamin(TEA)-Puffer (pH 7.5), 3 mM $MgCl_2$, 3 mM reduziertes Glutathion (GSH), 0.1 mM GTP, 1 mM 3-Isobutyl-1-methylxanthin (IBMX), geeignet verdünnte Enzymlösung sowie die Prüfsubstanz bzw. bei den Kontrollversuchen das Lösungsmittel. Die Prüfsubstanzen wurden in Dimethylsulfoxid (DMSO) gelöst und die Lösung mit DMSO/Wasser verdünnt, so daß die Endkonzentration an Prüfsubstanz in der Testlösung 50 $\mu$M betrug. Die DMSO-Konzentration in der Testlösung betrug 5 % (v/v). Die Reaktion wurde durch Zugabe der sGC gestartet. Der Reaktionsmix wurde für 15 bis 20 Minuten bei 37 °C inkubiert und dann durch Eiskühlung und Zugabe des Stop-Reagenz (50 mM EDTA, pH 8.0) gestoppt. Ein Aliquot von 50 $\mu$l wurde entnommen und zur Bestimmung des cGMP-Gehaltes mit dem Acetylierungs-Protokoll des Amersham-cGMP-EIA-Kits eingesetzt. Die Absorption der Proben wurde bei 450 nm (Referenz Wellenlänge 620 nm) in einem Mikrotiterplatten-Lesegerät gemessen. Die cGMP-Konzentration wurde über eine Eichkurve ermittelt, die unter denselben Versuchsbedingungen erhalten wurde. Die Aktivierung der sGC durch eine Prüfsubstanz wird angegeben als n-fache Stimulation der basalen Enzymaktivität, die bei den Kontrollversuchen (mit Lösungsmittel statt Prüfsubstanz) gefunden wurde (berechnet nach der Formel

$$\text{n-fache Stimulierung} = [\text{cGMP}]_{\text{Prüfsubstanz}} / [\text{cGMP}]_{\text{Kontrolle}} \quad ).$$

Es wurden folgende Werte ermittelt:

| Verbindung | Konzentration | n-fache Stimulierung |
|---|---|---|
| Beispiel 44 | 100 μM | 3.2-fach |

2) Relaxation der Ratten-Aorta

Für diesen Test wurden normotensive, männliche Wistar-Kyoto-Ratten durch einen Schlag auf das Genick getötet. Der Bauchraum und der Brustkorb wurden durch eine mittlere Sternotomie eröffnet. Anschließend wurde die deszendierende Aorta entnommen, von Bindegewebe befreit und in 8 Ringe von ca. 4 mm Länge geteilt. In das Lumen von 4 der 8 Ringe wurde eine Pinzettenspitze eingebracht. Durch vorsichtige, rollende Bewegungen der Ringe über die Pinzettenspitze wurde das Endothel entfernt. Alle 8 Aortenringe (4 mit Endothel und 4 ohne Endothel) wurden anschließend in ein Organbad (Schuler-Organbad; Hugo Sachs Elektronik) mit konstanter Temperatur von 37 °C zur isometrischen Messung des kontraktilen Tonus eingehängt. Die Ringe wurden 30 Minuten bei einer Ruhespannung von 1g in carbonierter (95 % $O_2$; 5 % $CO_2$) Krebs-Henseleit-Lösung (Zusammensetzung: $Na^+$ 144.0 mM; $K^+$ 5.9 mM; $Cl^-$ 126.9 mM; $Ca^{2+}$ 1.6 mM; $Mg^{2+}$ 1.2 mM; $H_2PO_4^-$ 1.2 mM; $SO_4^{2-}$ 1.2 mM; $HCO_3^-$ 25.0 mM; D-Glucose 11.1 mM) vom pH-Wert 7.4 kalibriert. Außerdem wurde der Krebs-Henseleit-LOsung 1 μmol/l Indomethacin zur Hemmung der Prostaglandin-Biosynthese zugegeben. Anschließend wurden die Ringe durch Zugabe von Phenylephrin (Konzentration in der Lösung: 1 μM) vorkontrahiert und die endothelabhängige Relaxation bzw. der funktionelle Verlust des Endothels wird durch Gabe von Acetylcholin (Konzentration in der Lösung: 1 μM) getestet. Im Anschluß an eine 30-minütige Waschperiode wurden die Ringe dann erneut durch Zusatz von Phenylephrin (1 μM) vorkontrahiert und durch Gabe von kumulativen Dosen der Prüfsubstanzen der Formel I wurde deren relaxierende Wirkung bestimmt. Die Auswertung der Daten erfolgte nach Standardverfahren. Angegeben wird die Konzentration $IC_{50}$, durch die die Kontraktion um 50 % gehemmt wird (50 %ige Relaxation).

**Patentansprüche**

1. Verbindung der Formel I,

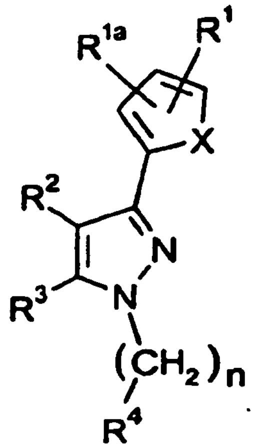

I

in der

X für O steht;
$R^1$ für einen ungesättigten Alkylrest mit bis zu 10 Kohlenstoffatomen steht, der eine oder zwei Doppelbindungen oder eine oder zwei Dreifachbindungen oder eine Doppelbindung und eine Dreifachbindung enthält und der durch Hydroxy substituiert ist;
$R^{1a}$ für Wasserstoff steht;
$R^2$ und $R^3$ zusammen mit den sie tragenden Kohlenstoffatomen einen Benzolring bilden, der unsubstituiert oder durch einen oder mehrere gleiche oder verschiedene Substituenten $R^5$ substituiert sein kann, wobei $R^5$ für

Halogen, ($C_1$-C3)-Alkyl, $CF_3$ oder ($C_1$-$C_3$-Alkyl-O- steht;
$R^4$ für Phenyl oder 5- oder 6-gliedriges Heteroaryl steht, die jeweils unsubstituiert oder durch einen oder mehrere Substituenten aus der Reihe Halogen, ($C_1$-$C_3$)-Alkyl und $CF_3$ substituiert sein können;
n für 0, 1 oder 2 steht;
in allen ihren stereoisomeren Formen und Mischungen davon in allen Verhältnissen, und ihre physiologisch verträglichen Salze.

**2.** Verbindung der Formel I gemäß Anspruch 1, in der der für $R^1$ stehende ungesättigte Alkylrest eine Doppelbindung enthält, in allen ihren stereoisomeren Formen und Mischungen davon in allen Verhältnissen, und ihre physiologisch verträglichen Salze.

**3.** Verbindung der Formel I gemäß Anspruch 1, in der der für $R^1$ stehende ungesättigte Alkylrest eine Dreifachbindung enthält, in allen ihren stereoisomeren Formen und Mischungen davon in allen Verhältnissen, und ihre physiologisch verträglichen Salze.

**4.** Verbindung der Formel I gemäß einem oder mehreren der Ansprüche 1 bis 3, in der der für $R^1$ stehende ungesättigte Alkylrest, der durch Hydroxy substituiert ist, ein Rest der Formel CH(OH)-Alk ist, worin Alk für einen ungesättigten Alkylrest mit bis zu 5 Kohlenstoffatomen steht, der eine Doppelbindung oder eine Dreifachbindung enthält, in allen ihren stereoisomeren Formen und Mischungen davon in allen Verhältnissen, und ihre physiologisch verträglichen Salze.

**5.** Verbindung der Formel I gemäß einem oder mehreren der Ansprüche 1 bis 4, in der der für $R^1$ stehende ungesättigte Alkylrest, der durch Hydroxy substituiert ist, ein Rest der Formel CH(OH)-Alk ist, worin Alk für einen Vinylrest, 2-Propenylrest, 2-Butenylrest, 3-Methyl-2-butenylrest, Ethinylrest, 2-Propinylrest oder 3-Butinylrest steht; in allen ihren stereoisomeren Formen und Mischungen davon in allen Verhältnissen, und ihre physiologisch verträglichen Salze.

**6.** Verbindung der Formel I gemäß einem oder mehreren der Ansprüche 1 bis 5, in der $R^2$ und $R^3$ zusammen mit den sie tragenden Kohlenstoffatomen einen unsubstituierten Benzolring bilden, in allen ihren stereoisomeren Formen und Mischungen davon in allen Verhältnissen, und ihre physiologisch verträglichen Salze.

**7.** Verfahren zur Herstellung einer Verbindung der Formel gemäß einem oder mehreren der Ansprüche 1 bis 6, **dadurch gekennzeichnet, daß** man

a) eine Verbindung der Formel VII mit einem Hydrazin der Formel III oder dessen Salz umsetzt,

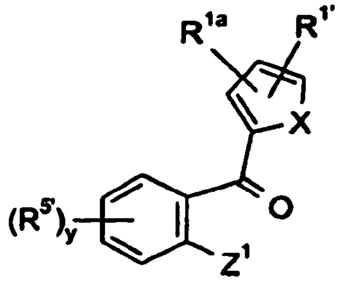

VII

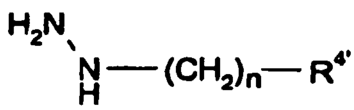

III

wobei in den Formeln VII und III die Reste X, $R^{1'}$, $R^{1a}$, $R^{4'}$ und $R^{5'}$ und die Zahl n die in den Ansprüchen 1 bis 6 angegebenen Bedeutungen von X, $R^1$, $R^{1a}$, $R^4$ und $R^5$ und n haben und zusätzlich in den Resten $R^{1'}$, $R^{4'}$ und $R^{5'}$ funktionelle Gruppen in geschützter Form oder in Form von Vorstufen vorliegen können, y für 0, 1, 2, 3 oder 4 steht und $Z^1$ für eine Abgangsgruppe steht, und anschließend gegebenenfalls die Reste $R^{1'}$, $R^{4'}$ und $R^{5'}$ in die Reste $R^1$, $R^4$ und $R^5$ mit den in den Ansprüchen 1 bis 6 angegebenen Bedeutungen überführt; oder
b) eine Verbindung der Formel VII mit einer Verbindung der Formel IX unter Abspaltung der Acylgruppe R-CO zu einer Verbindung der Formel X umsetzt und diese mit einer Verbindung der Formel XI alkyliert,

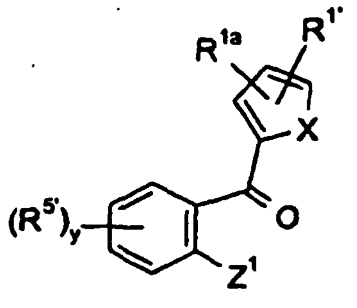

VII

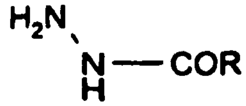

IX

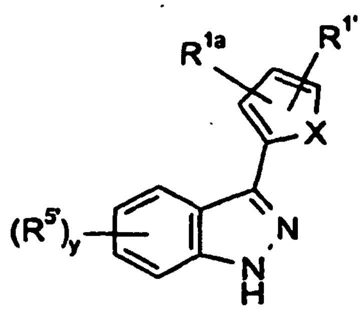

X

$$R^{4'}—(CH_2)_n—Z^2$$

XI

wobei in den Formeln VII, IX, X und XI die Reste X, $R^{1'}$, $R^{1a}$, $R^{4'}$ und $R^{5'}$, $Z^1$, n und y die unter a) angegebenen Bedeutungen haben, $Z^2$ für eine Abgangsgruppe steht und R für einen Alkylrest oder Arylrest steht, und anschließend gegebenenfalls die Reste $R^{1'}$, $R^{4'}$ und $R^{5'}$ in die Reste $R^1$, $R^4$ und $R^5$ mit den in den Ansprüchen 1 bis 6 angegebenen Bedeutungen überführt.

8. Verbindung der Formel I gemäß einem oder mehreren der Ansprüche 1 bis 6 und/oder ihr physiologisch verträgliches Salz zur Verwendung als Arzneimittel.

9. Pharmazeutisches Präparat, **dadurch gekennzeichnet, daß** es eine oder mehrere Verbindungen der Formel I gemäß einem oder mehreren der Ansprüche 1 bis 6 und/oder ihre physiologisch verträglichen Salze neben pharmazeutisch einwandfreien Trägerstoffen und/oder Zusatzstoffen enthält.

10. Verwendung einer Verbindung der Formel I gemäß einem oder mehreren der Ansprüche 1 bis 6 und/oder ihres physiologisch verträglichen Salzes zur Herstellung eines Medikaments zur Aktivierung der löslichen Guanylatcyclase.

11. Verwendung einer Verbindung der Formel I gemäß einem oder mehreren der Ansprüche 1 bis 6 und/oder ihres physiologisch verträglichen Salzes zur Herstellung eines Medikaments zur Therapie oder Prophylaxe von Herz-Kreislauf-Erkrankungen, endothelialer Dysfunktion, diastolischer Dysfunktion, Atherosklerose, Bluthochdruck, Angina pectoris, Thrombosen, Restenosen, Myocardinfarkt, Schlaganfällen, Herzinsuffizienz, Pulmonalhypertonie, erektiler Dysfunktion, Asthma bronchiale, chronischer Niereninsuffizienz, Diabetes oder Leberzirrhose oder zur Verbesserung einer eingeschränkten Lernfähigkeit oder Gedächtnisleistung.

**Claims**

1. A compound of the formula I

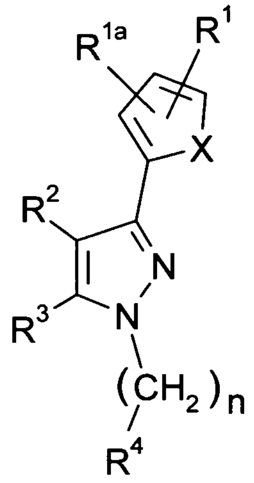

I

in which

X is O;
$R^1$ is an unsaturated alkyl radical having up to 10 carbon atoms which contains one or two double bonds or one or two triple bonds or one double bond and one triple bond and which is substituted by hydroxy;
$R^{1a}$ is hydrogen;
$R^2$ and $R^3$ together with the carbon atoms which carry them form a benzene ring which may be unsubstituted or substituted by one or more identical or different substituents $R^5$, where $R^5$ is halogen, $(C_1-C_3)$-alkyl, $CF_3$ or $(C_1-C_3)$-alkyl-O-;
$R^4$ is phenyl or 5- or 6-membered heteroaryl, each of which may be unsubstituted or substituted by one or more substituents from the group halogen, $(C_1-C_3)$-alkyl and $CF_3$;
n is 0, 1 or 2;

in all its stereoisomeric forms and mixtures thereof in all ratios, and its physiologically acceptable salts.

**2.** The compound of the formula I as claimed in claim 1, in which the unsaturated alkyl radical represented by $R^1$ contains a double bond, in all its stereoisomeric forms and mixtures thereof in all ratios, and its physiologically acceptable salts.

**3.** The compound of the formula I as claimed in claim 1, in which the unsaturated alkyl radical represented by $R^1$ contains a triple bond, in all its stereoisomeric forms and mixtures thereof in all ratios, and its physiologically acceptable salts.

**4.** The compound of the formula I as claimed in one or more of claims 1 to 3, in which the unsaturated alkyl radical represented by $R^1$, which is substituted by hydroxy, is a radical of the formula CH(OH)-Alk in which Alk is an unsaturated alkyl radical having up to 5 carbon atoms which contains a double bond or a triple bond, in all its stereoisomeric forms and mixtures thereof in all ratios, and its physiologically acceptable salts.

**5.** The compound of the formula I as claimed in one or more of claims 1 to 4, in which the unsaturated alkyl radical represented by $R^1$, which is substituted by hydroxyl, is a radical of the formula CH(OH)-Alk where Alk is a vinyl radical, 2-propenyl radical, 2-butenyl radical, 3-methyl-2-butenyl radical, ethynyl radical, 2-propynyl radical or 3-butynyl radical, in all its stereoisomeric forms and mixtures thereof in all ratios, and its physiologically acceptable salts.

**6.** The compound of the formula I as claimed in one or more of claims 1 to 5, in which $R^2$ and $R^3$ together with the carbon atoms which carry them form an unsubstituted benzene ring, in all its stereoisomeric forms and mixtures thereof in all ratios, and its physiologically acceptable salts.

**7.** A process for preparing a compound of the formula I as claimed in one or more of claims 1 to 6, which comprises

a) reacting a compound of the formula VII with a hydrazine of the formula III or a salt thereof,

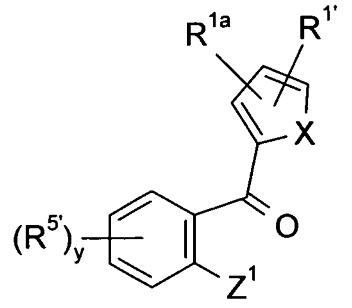

VII

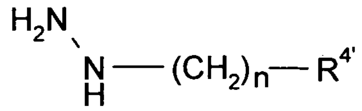

III

where in the formulae VII and III the radicals X, $R^{1'}$, $R^{1a}$, $R^{4'}$ and $R^{5'}$and the number n have the meanings of X, $R^1$, $R^{1a}$, $R^4$ and $R^5$ and n given in claims 1 to 6 and where additionally in the radicals $R^{1'}$, $R^{4'}$ and $R^{5'}$ functional groups may be present in protected form or in the form of precursors, y is 0, 1, 2, 3 or 4 and $Z^1$ is a leaving group, and subsequently, if appropriate, converting the radicals $R^{1'}$, $R^{4'}$and $R^{5'}$ into the radicals $R^1$, $R^4$ and $R^5$ which have the meanings given in claims 1 to 6; or

b) reacting a compound of the formula VII with a compound of the formula IX with elimination of the acyl group R-CO to give a compound of the formula X, and alkylating this compound with a compound of the formula XI,

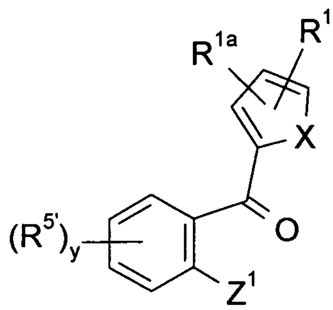

VII

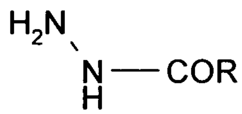

IX

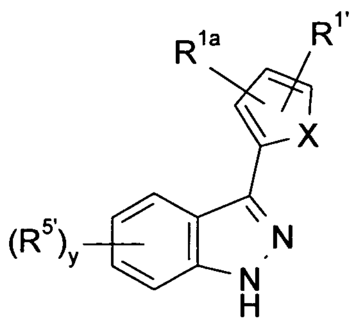

X

$$R^{4'}-(CH_2)_n-Z^2$$

XI

where in the formulae VII, IX, X and XI the radicals X, $R^{1'}$, $R^{1a}$, $R^{4'}$ and $R^{5'}$, $Z^1$, n and y have the meanings given under a), $Z^2$ is a leaving group and R is an alkyl radical or aryl radical, and subsequently, if appropriate, converting the radicals $R^{1'}$, $R^{4'}$ and $R^{5'}$ into the radicals $R^1$, $R^4$ and $R^5$ which have the meanings given in claims 1 to 6.

**8.** The compound of the formula I as claimed in one or more of claims 1 to 6 and/or its physiologically acceptable salt for use as a pharmaceutical.

**9.** A pharmaceutical preparation, which comprises one or more compounds of the formula I as claimed in one or more of claims 1 to 6 and/or its physiologically acceptable salts in addition to pharmaceutically acceptable carriers and/or additives.

**10.** The use of a compound of the formula I as claimed in one or more of claims 1 to 6 and/or its physiologically acceptable salt for preparing a medicament for the activation of soluble guanylate cyclase.

**11.** The use of a compound of the formula I as claimed in one or more of claims 1 to 6 and/or its physiologically acceptable salt for preparing a medicament for the therapy or prophylaxis of cardiovascular diseases, endothelial dysfunction, diastolic dysfunction, atherosclerosis, hypertension, angina pectoris, thromboses, restenoses, myocardial infarction, strokes, cardiac insufficiency, pulmonary hypertonia, erectile dysfunction, asthma bronchiale, chronic kidney insufficiency, diabetes or cirrhosis of the liver or for improving memory performance or a restricted ability to learn.

## Revendications

**1.** Composé de formule I

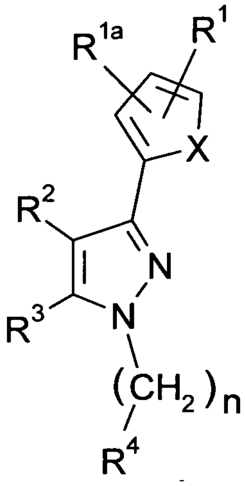

I

dans laquelle

X représente O ;
$R^1$ représente un radical alkyle insaturé comprenant jusqu'à 10 atomes de carbone, qui contient une ou deux doubles liaisons ou une ou deux triples liaisons ou une double liaison et une triple liaison et qui est substitué par hydroxy ;
$R^{1a}$ représente hydrogène ;
$R^2$ et $R^3$ forment ensemble avec les atomes de carbone qui les portent un cycle benzène, qui peut être non substitué ou substitué par un ou plusieurs substituants $R^5$ identiques ou différents, où $R^5$ représente halogène, $(C_1$-$C_3)$-alkyle, $CF_3$ ou $(C_1$-$C_3)$-alkyl-O- ;
$R^4$ représente phényle ou hétéroaryle à 5 ou 6 chaînons, qui peuvent être à chaque fois non substitués ou substitués par un ou plusieurs substituants de la série halogène, $(C_1$-$C_3)$-alkyle et $CF_3$ ;
n vaut 0, 1 ou 2 ;

dans toutes ses formes stéréo-isomères et leurs mélanges, dans tous les rapports, et ses sels physiologiquement acceptables.

**2.** Composé de formule I selon la revendication 1, dans laquelle le radical alkyle insaturé représentant $R^1$ contient une double liaison, dans toutes ses formes stéréo-isomères et leurs mélanges, dans tous les rapports, et ses sels physiologiquement acceptables.

**3.** Composé de formule I selon la revendication 1, dans laquelle le radical alkyle insaturé représentant $R^1$ contient une triple liaison, dans toutes ses formes stéréo-isomères et leurs mélanges, dans tous les rapports, et ses sels physiologiquement acceptables.

**4.** Composé de formule I selon l'une ou plusieurs des revendications 1 à 3, dans laquelle le radical alkyle insaturé représentant $R^1$, qui est substitué par hydroxy, représente un radical de formule CH(OH)-Alk, où Alk représente un radical alkyle insaturé comprenant jusqu'à 5 atomes de carbone, qui contient une double liaison ou une triple liaison,

dans toutes ses formes stéréo-isomères et leurs mélanges, dans tous les rapports, et ses sels physiologiquement acceptables.

**5.** Composé de formule I selon l'une ou plusieurs des revendications 1 à 4, dans laquelle le radical alkyle insaturé représentant $R^1$, qui est substitué par hydroxy, représente un radical de formule CH(OH)-Alk, où Alk représente un radical vinyle, 2-propényle, 2-butényle, 3-méthyl-2-butényle, éthynyle, 2-propynyle ou 3-butynyle, dans toutes ses formes stéréo-isomères et leurs mélanges, dans tous les rapports, et ses sels physiologiquement acceptables.

**6.** Composé de formule I selon l'une ou plusieurs des revendications 1 à 5, dans laquelle $R^2$ et $R^3$, ensemble avec les atomes de carbone qui les portent, forment un cycle benzène non substitué, dans toutes ses formes stéréo-isomères et leurs mélanges, dans tous les rapports, et ses sels physiologiquement acceptables.

**7.** Procédé pour la préparation d'un composé de formule I selon l'une ou plusieurs des revendications 1 à 6, **caractérisé en ce que**

a) on transforme un composé de formule VII avec une hydrazine de formule III ou son sel

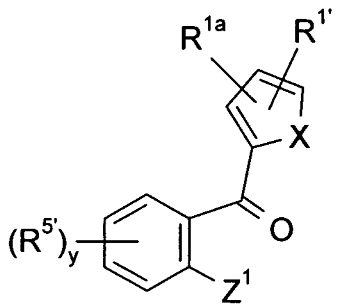

VII

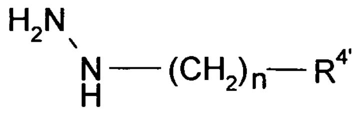

III

où, dans les formules VII et III, les radicaux X, $R^{1'}$, $R^{1a}$, $R^{4'}$ et $R^{5'}$ et le nombre n ont les significations indiquées dans les revendications 1 à 6 de X, $R^1$, $R^{1a}$, $R^4$ et $R^5$ et n et en outre, les groupes fonctionnels dans les radicaux $R^{1'}$, $R^{4'}$ et $R^{5'}$ peuvent se trouver sous forme de groupes protégés ou sous forme de précurseurs, y vaut 0, 1, 2, 3 ou 4 et $Z^1$ représente un groupe partant, puis on transforme le cas échéant les radicaux $R^{1'}$, $R^{4'}$ et $R^{5'}$ en radicaux $R^1$, $R^4$ et $R^5$ ayant les significations indiquées dans les revendications 1 à 6 ; ou

b) on transforme un composé de formule VII avec un composé de formule IX avec dissociation du groupe acyle R-CO en un composé de formule X et on alkyle celui-ci avec un composé de formule XI,

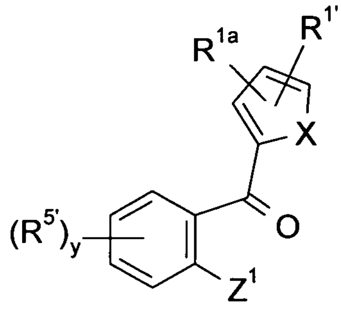

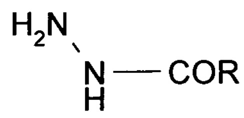

IX

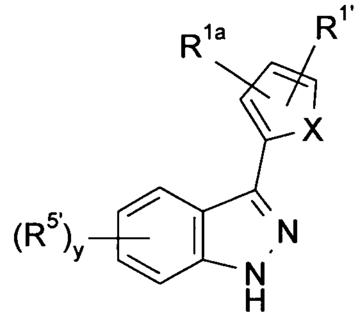

VII

X

$$R^{4'}-(CH_2)_n-Z^2$$

XI

où, dans les formules VII, IX, X et XI, les radicaux X, $R^{1'}$, $R^{1a}$, $R^{4'}$ et $R^{5'}$, $Z^1$, n et y ont les significations indiquées dans a), $Z^2$ représente un groupe partant et R représente un radical alkyle ou aryle, puis on transforme le cas échéant les radicaux $R^{1'}$, $R^{4'}$ et $R^{5'}$ en radicaux $R^1$, $R^4$ et $R^5$ ayant les significations indiquées dans les revendications 1 à 6.

**8.** Composé de formule I selon l'une ou plusieurs des revendications 1 à 6 et/ou son sel physiologiquement acceptable destiné à une utilisation comme médicament.

**9.** Préparation pharmaceutique, **caractérisée en ce qu'**elle contient un ou plusieurs composés de formule I selon l'une ou plusieurs des revendications 1 à 6 et/ou ses/leurs sels physiologiquement acceptables, en plus de substances support et/ou d'additifs pharmaceutiquement acceptables.

**10.** Utilisation d'un composé de formule I selon l'une ou plusieurs des revendications 1 à 6 et/ou son sel physiologiquement acceptable pour la préparation d'un médicament destiné à l'activation de la guanylate-cyclase soluble.

**11.** Utilisation d'un composé de formule I selon l'une ou plusieurs des revendications 1 à 6 et/ou son sel physiologiquement acceptable pour la préparation d'un médicament destiné à la thérapie ou la prophylaxie de maladies cardiovasculaires, d'un dysfonctionnement endothélial, d'un dysfonctionnement diastolique, de l'athérosclérose, de l'hypertension, de l'angine de poitrine, de thromboses, de resténoses, de l'infarctus du myocarde, d'attaques, de l'insuffisance cardiaque, de l'hypertonie pulmonaire, d'un dysfonctionnement érectile, de l'asthme bronchique, de l'insuffisance rénale chronique, du diabète ou de la cirrhose du foie ou pour l'amélioration d'une aptitude à l'apprentissage ou d'une mémoire limitées.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 667345 A


### In der Beschreibung aufgeführte Nicht-Patentliteratur


- **D. L. Veseley.** *Eur. J. Clin. Invest.,* 1985, vol. 15, 258
- **D. L. Veseley.** *Biochem. Biophys. Res. Comm.,* 1979, vol. 88, 1244
- **Ignarro et al.** *Adv. Pharmacol.,* 1994, vol. 26, 35
- **Pettibone et al.** *Eur. J. Pharmacol.,* 1985, vol. 116, 307
- **Yu et al.** *Brit. J. Pharmacol.,* 1995, vol. 114, 1587
- **Ko et al.** *Blood,* 1994, vol. 84, 4226
- **Yu et al.** *Biochem. J.,* 1995, vol. 306, 787
- **Teng et al.** *Brit. J. Pharmacol.,* 1995, vol. 116, 1973
- **Houben-Weyl.** Methoden der Organischen Chemie. Thieme-Verlag
- Organic Reactions. John Wiley & Sons
- *Methods in Enzymology,* vol. 195, 377